# EUROPEAN PATENT SPECIFICATION

(11) **EP 3 220 927 B1**
(45) Date of publication and mention of the grant of the patent: **05.01.2022**
(21) Application number: 15860628.5
(22) Date of filing: 20.11.2015
(51) Int. Cl.: C07K 16/28

(54) **SYSTEMS AND METHODS FOR ASSESSING MODULATORS OF IMMUNE CHECKPOINTS**
SYSTEME UND VERFAHREN ZUR BEURTEILUNG VON MODULATOREN VON IMMUNPRÜFPUNKTEN
SYSTÈMES ET PROCÉDÉS POUR ÉVALUER DES MODULATEURS DE POINTS DE CONTRÔLE IMMUNITAIRES

(30) Priority: 20.11.2014 US 201462082458 P
(43) Date of publication of application: 27.09.2017
(60) Divisional application: 21199893.5
(73) Proprietor: Promega Corporation, Madison, WI 53711-5399 (US)
(72) Inventor: CONG, Mei, Madison, Wisconsin 53711-5399 (US); CHENG, Zhijie Jey, Madison, Wisconsin 53711-5399 (US); KARASSINA, Natasha, Madison, Wisconsin 53711-5399 (US); GRAILER, Jamison, Madison, Wisconsin 53711-5399 (US); FAN, Frank, Madison, Wisconsin 53711-5399 (US)
(74) Representative: Potter Clarkson
(86) International application number: PCT/US2015/061906
(87) International publication number: WO 2016/081854

(56) References cited:
- WO-A1-2015/112800
- WO-A2-03/042402
- US-A1- 2005 100 897
- US-A1- 2006 034 810
- US-A1- 2010 028 450
- Zhi-Jie Jey Cheng ET AL: "Abstract 5440: Novel PD-1 blockade bioassay to assess therapeutic antibodies in PD-1 and PD-L1 immunotherapy programs | Cancer Research", Experimental and Molecular Therapeutics, 22 April 2015 (2015-04-22), XP055458898, Philadelphia, USA Retrieved from the Internet: URL:http://cancerres.aacrjournals.org/cont ent/75/15_Supplement/5440 [retrieved on 2018-03-13]
- ZHI-JIE JEY CHENG ET AL: "Abstract 5440: Novel PD-1 blockade bioassay to assess therapeutic antibodies in PD-1 and PD-L1 immunotherapy programs", CANCER RESEARCH, vol. 75, no. 15 Supplement, 1 August 2015 (2015-08-01), pages 5440-5440, XP055458900, US ISSN: 0008-5472, DOI: 10.1158/1538-7445.AM2015-5440
- Ali El Baya: "Immunotherapy Screening - A focus on PD-1/PD-L1/PD-L2 Pathway in drug discovery", , 7 July 2014 (2014-07-07), XP055458888, Retrieved from the Internet: URL:https://www.tebu-bio.com/blog/2014/07/ 07/immunotherapy-screening-part-2-pd-1pd-l 1pd-l2-pathway/ [retrieved on 2018-03-13]
- Ali El Baya: "Immunotherapy Screening - B7-1 / CD28 and B7-1 / CTLA4 Pathways in Drug Discovery", , 1 July 2014 (2014-07-01), XP055458891, Retrieved from the Internet: URL:https://www.tebu-bio.com/blog/2014/07/ 01/immunotherapy-screening-b7-1cd28-and-b7 -1ctla4-pathways-in-drug-discovery/ [retrieved on 2018-03-13]
- LEE ET AL.: 'Down-regulation of interleukin-2 production by CD 4(+) T cells expressing TIM-3 through suppression of NFAT dephosphorylation and AP -1 transcription' IMMUNOBIOLOGY vol. 217, no. 10, 16 January 2012, pages 986 - 995, XP055443307 DOI: 10.1016/J.IMBIO.2012.01.012
- CHOW ET AL.: 'Requirement for transcription factor NFAT in interleukin-2 expression' MOL CELL BIOL. vol. 19, no. 3, 01 March 1999, pages 2300 - 2307, XP055443309 DOI: 10.1128/MCB.19.3.2300
- None

## Description

Described herein are compositions, systems, and methods for assessing modulators of immune checkpoints. In particular, artificial antigen presenting cells (aAPCs) and immune effector cells are provided to assess the potency of test agents to inhibit immune checkpoints.

### BACKGROUND

Among the most promising approaches to activating therapeutic antitumor immunity is the blockade of immune checkpoints. Immune checkpoints refer to a plethora of inhibitory pathways hardwired into the immune system that are crucial for maintaining self-tolerance and modulating the duration and amplitude of physiological immune responses. It is now clear that tumors co-opt certain immune-checkpoint pathways as a major mechanism of immune resistance, particularly against T cells that are specific for tumor antigens. Because many of the immune checkpoints are initiated by ligand-receptor interactions, they can be blocked by agents that inhibit these interactions, for example antibodies specific for the ligand or receptor.

A ligand-receptor interaction that has been investigated as a target for cancer treatment is the interaction between the transmembrane programmed cell death 1 protein (PD-1; also known as CD279) and its ligand, PD-1 ligand 1 (PD-L1) and PD-1 ligand 2 (PD-L2). In normal physiology, PD-L1 and PD-L2 on the surface of a cell binds to PD-1 on the surface of an immune cell, which inhibits the activity of the immune cell. Upregulation of PD-L1 on the surface of some cancer cells may allow them to evade the host immune system by inhibiting T cells that might otherwise attack tumors. Antibodies that bind to either PD-1 or PD-L1, and therefore block the interaction, defeat the inhibitory mechanism and allow the immune cells to attack the cancer cells or tumor. Initial clinical trial results with an IgG4 PD-1 antibody called NIVOLUMAB have been published (Pardoll, DM (Mar 22, 2012). "The blockade of immune checkpoints in cancer immunotherapy." Nature reviews cancer. 12 (4): 252-64).

US2006/0034810 discloses systmes comprising an effector cell expressing PD-1 and an antigen presenting cell expressing PD-L1 or PD-L2. The artificial antigen presenting cell of D7 displays on its surface a TCR activator and an immune checkpoint ligand. The APC may comprise i.a. CD80, PD-L1, PD-L2 or HVEM.

Traditional methods of measuring antibodies against immunoblockade receptors have numerous drawbacks. For example, animal models can be used to assess the antitumor effects of mAb blockage by phenotypic analysis of tumor masses in tumor-bearing mice, mean survival time, overall survival rate of mice, etc. Such analysis has the major drawbacks of being cost and time prohibitive. As an alternative, freshly isolated peripheral blood mononuclear cells (PBMC) can be used to study immunotherapy drugs on inhibitory blockade of cytokine production. For example, cryopreserved PBMCs have been used in clinical studies, which reported with significantly reduced expression of both PD-1 and PD-L1 on PBMC-derived CD3+/CD8+ T cells and CD45+/CD14+ monocytes obtained from adult control subjects. However, the isolation of PBMCs is tedious and such experiments produce great variability in results. Finally, cell-based assays have been developed to measure IL-2 production (ELISA based assay) in Jurkat cells over expressing immune checkpoint receptors, however such assays require a minimum of two days after antibody stimulation to perform. Convenient methods for measuring the effectiveness of antibodies against immune checkpoint receptors are needed.

### SUMMARY

The invention is as described in the Claims.

Described herein are compositions, systems, and methods for assessing modulators of immune checkpoints. In particular, artificial antigen presenting cells (aAPCs) and immune effector cells are provided to assess the potency of test agents (e.g., antibodies) to inhibit immune checkpoints.

In some options, described herein are compositions comprising an artificial antigen presenting cell (aAPC) or phospholipid droplet, displaying on its surface: (1) an antigen responsive element activator, and (2) an immune checkpoint ligand (ICL). In some options, the antigen responsive element activator is a T cell receptor (TCR) complex activator. In some options, compositions are provided comprising an artificial antigen presenting cell (aAPC) or phospholipid droplet displaying on its surface a T cell receptor (TCR) activator and an immune checkpoint ligand (ICL). Although options described herein are typically described in reference to the TCR complex and a TCR activator, the compositions, systems, and methods described herein may also be applied to other antigen responsive elements and activators thereof. In some options, the aAPC further displays on its surface a second (or third, fourth, etc.), different immune checkpoint ligand.

In some options, TCR activator is a surface displayed molecular entity (e.g., protein, peptide, polypeptide, etc.) that binds to, or otherwise interacts with a component of the TCR complex to activate the TCR complex and TCR-dependent pathways. In some options, the TCR activator is an anti-cluster of differentiation 3 antibody (anti-CD3), a membrane fusion protein containing an antibody fragment of anti-CD3, or antibody fragment thereof, major histocompatibility complex (MHC), etc.

In some options, the ICL is a molecular entity (e.g., protein, peptide, polypeptide, etc.) that interacts with an immune checkpoint receptor (ICR) on an effector cell (e.g., T cell, Jurkat, etc.) to modulate (e.g., inhibit, enhance, etc.) the immune response of the effector cell. In some options, the ICL is any suitable immune checkpoint ligand including but not limited to, PD-L1, PD-L2, B7-H4, CD155, galectin-9, HVEM, etc.

In some options, described herein are compositions comprising an artificial antigen presenting cell (aAPC) displaying on its surface: (1) A TCR activator (e.g., anti-CD3) and (2) an immune checkpoint ligand (e.g., PD-L1).

In some options, the artificial, exogenous, and/or engineered elements of an APC include, but are not limited to: a TCR activator (e.g., anti-CD3 antibody, MHC, etc.), an immune checkpoint ligand (e.g., PD-L1), etc.

In some options, described herein are compositions comprising artificial antigen presenting cells (aAPCs) or phospholipid droplets displaying on their surface a T cell receptor (TCR) activator and an immune checkpoint ligand (ICL). In some options, upon interaction of the aAPC or phospholipid droplet with an effector cell comprising a TCR and an immune checkpoint receptor (ICR), the TCR activator activates the TCR of the effector cell, and the ICL interacts with the ICR forming an ICR/ICL complex. In some options, formation of the ICR/ICL complex modulates the downstream effects of TCR activation. In some options, the TCR activator is an anticluster-of-differentiation-3 (CD3) antibody, or antibody fragment thereof, or major histocompatibility complex (MHC). In some options, the ICL is selected from the group consisting of PD-L1, PD-L2, B7-H4, CD155, galectin-9, and HVEM.

In some options, described herein are systems comprising: (a) an artificial antigen presenting cell or phospholipid droplet displaying on its surface: (i) a T cell receptor (TCR) activator and (ii) an immune checkpoint ligand (ICL); and (b) an artificial effector cell displaying on its surface an immune checkpoint receptor (ICR) and comprising: (i) a TCR complex and (ii) a reporter (e.g., a TCR-pathway-dependent reporter) of one or more of: (A) ICR/ICL complex formation, (B) TCR activation, and/or (C) the TCR activation pathway-dependent reporter. In some options, formation of an ICR/ICL complex results in a detectable alteration in signal (e.g., change in activity, change in expression, etc.) from the TCR-activation-pathway-dependent reporter (e.g., versus in the absence of ICR/ICL interaction). In some options, systems further comprise a blockade agent or test blockade agent. In some options, the blockade agent or test blockade agent inhibits formation of the ICR/ICL complex resulting in inhibition of the signal from the TCR activation pathway-dependent reporter. In some options, the alteration of the signal from the TCR activation pathway-dependent reporter is at least 2-fold (e.g., 2-fold, 3-fold, 4-fold, 5-fold, 6-fold, 7-fold, 8-fold, 9-fold, 10-fold, 11-fold, 12-fold, 13-fold, 14-fold, 15-fold, 20-fold, 50-fold, 100-fold, 200-fold, 500-fold, 1000-fold, 2000-fold, 5000-fold, and any ranges therein).

In some options, the TCR activator is a surface displayed molecular entity (e.g., protein, peptide, polypeptide, etc.) that binds to, or otherwise interacts with, a component of the TCR complex to active the TCR complex and TCR-dependent pathways. In some options, the TCR activator is anti-cluster of differentiation 3 antibody (anti-CD3) or antibody fragment thereof, major histocompatibility complex (MHC), etc.

In some options, the ICL is a molecular entity (e.g., protein, peptide, polypeptide, etc.) that interacts with an immune checkpoint receptor (ICR) on an effector cell (e.g., T cell, Jurkat, etc.) to modulate (e.g., inhibit, enhance, etc.) the immune response of the effector cell. In some options, the ICL is any suitable immune checkpoint ligand including, but not limited to, PD-L1, PD-L2, B7-H4, CD155, galectin-9, HVEM, etc.

In some options, the artificial effector cell is a T cell selected from the group including, but not limited to, Jurkat cells, HuT-78, CEM, Molt-4, etc., that has been engineered to contain, express, etc., one or more elements (e.g., a reporter construct, exogenous gene, etc.) not native to the parent (e.g., non-artificial) cell.

In some options, the ICR is any suitable immune inhibitory receptor including, but not limited to, PD-1, CTLA-4, LAG-3, TIM-3, TIGIT, CD160, BTLA, IL-10 receptor, etc.

In some options, the TCR complex is an octomeric complex of variable TCR receptor α and β chains with three dimeric signaling modules CD3δ/ε, CD3γ/ε, and CD247 ζ/ζ or ζ/η.

In some options, the reporter (e.g., TCR activation pathway-dependent reporter) is an element on or within an effector cell having a characteristic (e.g., activity, expression, concentration, localization, interactions, modification, etc.) which is dependent upon, or correlates with, one or more of (i) ICR/ICL complex formation, (ii) TCR activation, and/or (iii) activation of the TCR signaling pathway. In some options, the reporter is downstream of ICR/ICL complex formation and/or TCR activation (e.g., an engineered reporter construct, a detectable element/signal/interaction naturally occurring within said effector cell). In some options, a reporter is an element on or within the effector cell having a characteristic (e.g., activity, expression, concentration, localization, interactions, modification, etc.) which is altered by one or more of (i) ICR/ICL complex formation, (ii) TCR activation, and/or (iii) the TCR signaling pathway. In some options, the reporter is intrinsic to the effector cell (e.g., an intrinsic element of T cells, Jurkat cells, etc.). In such options, alterations in characteristics of that intrinsic reporter are detectable as a signal of the presence, absence, and or level of one or more of i) ICR/ICL complex formation, (ii) TCR activation, and/or (iii) the TCR signaling pathway activation. Exemplary intrinsic reporters and/or signals include, but are not limited to: gene expression (e.g., of an TCR-pathway-dependent gene), protein concentration (e.g., a protein expressed by a TCR-pathway-dependent gene), a protein-protein interaction (e.g., mediated by ICR/ICL complex formation and/or TCR activation), activity of a TCR-dependent protein (e.g., an enzyme, proteinase, kinase), localization and movement of an intrinsic effector cell element, etc. In some options, the reporter is an extrinsic to the effector cell (e.g., a reporter element or construct that has been introduced or engineered into the effector cell). In such options, alterations in a characteristic of the extrinsic and/or engineered reporter are detectable as a signal of the presence, absence, and or level of one or more of: (i) ICR/ICL complex formation, (ii) TCR activation, and/or (iii) the TCR signaling pathway activation. Exemplary extrinsic reporters and/or signals include, but are not limited to: expression of a TCR-dependent and/or TCR-pathway-dependent reporter construct (e.g., under the control of an NFAT-response element, NF-κB-response element, AP1-response element, a promoter containing one or multiple of the mentioned response elements, IL-2 promoter), activity of a reporter whose activation and/or expression is TCR- or TCR-pathway dependent (e.g., luciferase, beta lactamase, CAT, SEAP, fluorescent protein, etc.), a protein-protein interaction, protein movement, endogenous gene up-regulation detection via qPCR, etc.

In some options, describedherein are systems comprising: (a) an artificial antigen presenting cell or phospholipid droplet displaying on its surface: (i) a T cell receptor (TCR) activator, (ii) a first immune checkpoint ligand (ICL), and (iii) a second ICL; and (b) an artificial effector cell displaying on its surface: (i) a first immune checkpoint receptor (ICR), (ii) a second ICR, and (iii) a TCR complex, and comprising a reporter (e.g., a TCR-pathway-dependent reporter) of one or more of: (A) complex formation between the first ICR and the first ICL, (B) complex formation between the second ICR and the second ICL, (C) TCR activation, and/or (D) the TCR activation pathway-dependent reporter. In some options, formation of a complex between the first ICR and first ICL and/or second ICR and second ICL results in a detectable alteration in signal (e.g., change in activity, change in expression, etc.) from the TCR-activation-pathway-dependent reporter (e.g., versus in the absence of an ICR/ICL interaction). In some options, systems further comprise a blockade agent or test blockade agent. In some options, the blockade agent or test blockade agent inhibits formation of the complex between the first ICR and first ICL and/or second ICR and second ICL resulting in inhibition of the signal from the TCR activation pathway-dependent reporter. In some options, the alteration of the signal from the TCR activation pathway-dependent reporter is at least 2-fold (e.g., 2-fold, 3-fold, 4-fold, 5-fold, 6-fold, 7-fold, 8-fold, 9-fold, 10-fold, 11-fold, 12-fold, 13-fold, 14-fold, 15-fold, 20-fold, 50-fold, 100-fold, 200-fold, 500-fold, 1000-fold, 2000-fold, 5000-fold, and any ranges therein).

In some options, described herein are systems comprising: (a) an effector cell displaying T cell Receptor (TCR) and an immune checkpoint receptor (ICR) on its surface, and a TCR activation pathway-dependent reporter (e.g., nuclear factor of activated T cells (NFAT) promoter-dependent reporter); and (b) an artificial antigen presenting cell (aAPC) displaying a TCR activator (e.g., surface expressed anti-cluster of differentiation 3 antibody or antibody fragments, major histocompatibility complex (MHC), etc.) and an immune checkpoint ligand (ICL); wherein the ICR and ICL form an ICR/ICL complex upon interaction. In some options, formation of the ICR/ICL complex results in a suppressed expression of the NFAT-promoter-dependent reporter. In some options, systems further comprise a blockade agent or test blockade agent. In some options, the blockade agent or test blockade agent inhibits formation of the ICR/ICL complex, resulting in an increased expression of the NFAT-promoter-dependent reporter. In some options, increased expression is at least 2-fold (e.g., 2-fold, 3-fold, 4-fold, 5-fold, 6-fold, 7-fold, 8-fold, 9-fold, 10-fold, 11-fold, 12-fold, 13-fold, 14-fold, 15-fold, 20-fold, 50-fold, 100-fold, 200-fold, 500-fold, 1000-fold, 2000-fold, 5000-fold, and any ranges therein). In some options, increased expression is a 6-12 fold increase over suppressed expression. In some options, the effector cell is a T cell including, but not limited to, Jurkat cells, HuT-78, CEM, Molt-4, etc. In some options, the reporter is a luciferase, a beta lactamase, CAT, SEAP, a fluorescent protein, a gene product (e.g., detected by qPCR, mass spectrometry, etc.), etc. In some options, the ICR is any immune inhibitory receptor including but not limited to PD-1, CTLA-4, LAG-3, TIM-3, TIGIT, CD160, BTLA, IL-10 receptor, etc., and the ICL is any corresponding immune inhibitory ligand including, but not limited to, PD-L1, PD-L2, B7-H4, CD155, galectin-9, HVEM, etc. In some options, the blockade agent or test blockade agent is an antibody (or antibody fragment), protein, peptide, or small molecule, vaccine, or combination thereof.

In some options, described herein are systems comprising: (a) an effector cell comprising TCR and PD-1 on its surface and a TCR activation pathway-dependent (e.g., NFAT-promoter-dependent) reporter; and (b) an artificial antigen presenting cell displaying a TCR activator (e.g., surface expression of anti-cluster of differentiation 3 antibody or antibody fragments, major histocompatibility complex (MHC), etc.) and PD-L1 on its surface. In some options, formation of a PD-1/PD-L1 complex results in a suppressed expression of the TCR activation pathway-dependent (e.g., NFAT-promoter-dependent) reporter. In some options, systems further comprise a blockade agent or test blockade agent. In some options, the blockade agent or test blockade agent inhibits formation of the PD-1/PD-L1 complex, resulting in an increased expression of the TCR activation pathway-dependent reporter. In some options, increased expression is at least 2-fold (e.g., 2-fold, 3-fold, 4-fold, 5-fold, 6-fold, 7-fold, 8-fold, 9-fold, 10-fold, 11-fold, 12-fold, 13-fold, 14-fold, 15-fold, 20-fold, 50-fold, 100-fold, 200-fold, 500-fold, 1000-fold, 2000-fold, 5000-fold, and any ranges therein).

In some options, the reporter is a luciferase, a beta lactamase, CAT, SEAP, a fluorescent protein, a protein-protein interaction, etc. In some options, expression is detected by directly measuring the amount of gene expression (e.g., by qPCR, by mass spectrometry, etc.).

In some options, described herein are compositions comprising an artificial effector cell displaying a TCR and any immune inhibitory receptor including, but not limited to, PD-1, CTLA-4, LAG-3, TIM-3, TIGIT, CD160, BTLA, IL-10 receptor, etc., on its surface and comprising a TCR activation pathway-dependent reporter (e.g., NFAT-promoter-dependent reporter) such as a luciferase, a beta lactamase, CAT, SEAP, a fluorescent protein, etc.

In some options, described herein are systems comprising: (a) an effector cell displaying on its surface an immune checkpoint receptor (ICR) and comprising a T cell receptor (TCR); and (b) an artificial antigen presenting cell (aAPC) displaying on its surface a T cell receptor (TCR) activator and an immune checkpoint ligand (ICL); wherein the ICR and ICL form an ICR/ICL complex upon interaction. In some options, formation of the ICR/ICL complex results in modulation of TCR activation by the TCR activator and/or modulation of one or more TCR-dependent pathways. In some options, modulation comprises: (a) inhibition of TCR activation by the TCR activator and/or one or more TCR-dependent pathways; or (b) enhancement of TCR activation by the TCR activator and/or one or more TCR-dependent pathways. In some options, the TCR activator is an anti-cluster-of-differentiation-3 (CD3) antibody, or antibody fragment thereof, or major histocompatibility complex (MHC). In some options, the ICL is selected from the group consisting of PD-L1, PD-L2, B7-H4, CD155, galectin-9, and HVEM. In some options, the ICR is selected from the group consisting of PD-1, CTLA-4, LAG-3, TIM-3, CD160, TIGIT, IL-10 receptor, and BTLA. In some options, the effector cell is selected from the group consisting of a Jurkat, HuT-78, CEM, and Molt-4. In some options, the effector cell further comprises a reporter of: TCR activation, TCR pathway activation, and/or ICR/ICL complex modulation of TCR activation or TCR pathway activation. In some options, the reporter characteristic comprises: cellular concentration, expression, activity, localization, or protein-protein interactions. In some options, formation of the ICR/ICL complex modulates said characteristic. In some options, the reporter is a natural reporter, e.g., intrinsic to the effector cell type, having a characteristic which is detectable and correlates to TCR activation, TCR pathway activation, and/or ICR/ICL complex modulation of TCR activation or TCR pathway activation. In some options, the reporter is an artificial reporter, e.g., exogenous to the effector cell type, having a characteristic which is detectable and correlates to TCR activation, TCR pathway activation, and/or ICR/ICL complex modulation of TCR activation or TCR pathway activation. In some options, the reporter is a gene, the expression of which is under the control of TCR-pathway-dependent reporter. In some options, the TCR-pathway-dependent reporter is a nuclear factor of activated T cells (NFAT) promoter. In some options, the reporter comprises a gene coding for a protein selected from the group consisting of a luciferase, a beta lactamase, CAT, SEAP, a fluorescent protein, or a quantifiable gene product. In some options, the system further comprises a blockade agent or test blockade agent. In some options, the blockade agent inhibits formation of the ICR/ICL complex, resulting in modulation of TCR activation or TCR pathway activation. In some options, the blockade agent or test blockade agent is a small molecule, peptide, protein, or antibody.

In some options, described herein are systems comprising: (a) an effector cell comprising TCR and PD-1 on its surface and an NFAT-promoter-dependent luciferase; and (b) an artificial antigen presenting cell (aAPC) displaying on its surface: anti-CD3 antibody or antibody fragment thereof and PD-L1. In some options, formation of a PD-1/PD-L1 complex results in a suppressed expression of the NFAT-promoter-dependent luciferase. In some options, systems further comprise a blockade agent or test blockade agent. In some options, the blockade agent or test blockade agent inhibits formation of the PD-1/PD-L1 complex resulting in an increased expression of the NFAT-promoter-dependent luciferase.

In some options, described herein are methods comprising: (a) contacting: (i) an artificial antigen presenting cell (aAPC) displaying on its surface: (A) a T cell receptor (TCR) activator and (B) an immune checkpoint ligand (ICL) with (ii) an effector cell comprising a TCR complex and displaying on its surface an immune checkpoint receptor (ICR), wherein interaction of the TCR activator with the TCR complex activates a TCR signaling pathway, wherein said ICR and said ICL form an ICR/ICL complex upon interaction, and wherein formation of the ICR/ICL complex enhances or inhibits TCR activation and/or the TCR signaling pathway; and (b) detecting the presence, absence, and/or level of TCR activation in said effector cell. In some options, the methods further comprise: (c) contacting said effector cell and/or said aAPC with a blockade agent or test blockade agent. In some options, the methods further comprise: (d) detecting the effect of said blockade agent or test blockade agent on (i) ICR/ICL complex formation, (ii) TCR activation, and/or (iii) the TCR signaling pathway. In some options, one or more of (i) ICR/ICL complex formation, (ii) TCR activation, and/or (iii) the TCR signaling pathway are detected in the presence and absence of said blockade agent or test blockade agent to determine an effect.

In some options, (i) ICR/ICL complex formation, (ii) TCR activation, and/or (iii) the TCR signaling pathway are detected based upon a reporter downstream of ICR/ICL complex formation and/or TCR activation (e.g., an engineered reporter construct, a detectable element/signal/interaction naturally occurring within said effector cell). In some options, a reporter is an element on or within the effector cell having a characteristic (e.g., activity, expression, concentration, localization, interactions, modification, etc.) of which is altered by one or more of (i) ICR/ICL complex formation, (ii) TCR activation, and/or (iii) the TCR signaling pathway. In some options, the reporter is intrinsic to the effector cell (e.g., an intrinsic element of T cells, Jurkat cells, etc.). In such options, alterations in a characteristic of that intrinsic reporter are detectable as a signal of the presence, absence, and or level of one or more of i) ICR/ICL complex formation, (ii) TCR activation, and/or (iii) the TCR signaling pathway activation. Exemplary intrinsic reporters and/or signals include, but are not limited to: gene expression (e.g., of a TCR-pathway-dependent gene), protein concentration (e.g., a protein expressed by a TCR-pathway-dependent gene), a protein-protein interaction (e.g., mediated by ICR/ICL complex formation and/or TCR activation), TCR-pathway-dependent protein modification, activity of a TCR-dependent protein, localization of an intrinsic effector cell element, etc. In some options, the reporter is extrinsic to the effector cell (e.g., a reporter element or construct that has been introduced or engineered into the effector cell). In such options, alterations in characteristics of the extrinsic and/or engineered reporter are detectable as a signal of the presence, absence, and or level of one or more of: (i) ICR/ICL complex formation, (ii) TCR activation, and/or (iii) the TCR signaling pathway activation. Exemplary extrinsic reporters and/or signals include, but are not limited to: expression of a TCR-dependent and/or TCR-pathway-dependent reporter construct (e.g., under the control of an NFAT promoter), activity of a reporter whose activation and/or expression is TCR- or TCR-pathway dependent (e.g., luciferase, beta lactamase, CAT, SEAP, fluorescent protein, etc.), a protein-protein interaction, protein movement, detection of protein modification, endogenous gene up-regulation detection via qPCR, etc.

In some options, the TCR activator is a surface displayed molecular entity (e.g., protein, peptide, polypeptide, etc.) that binds to, or otherwise interacts with, a component of the TCR complex to activate the TCR complex and TCR-dependent pathways. In some options, the TCR activator is anti-cluster of differentiation 3 antibody (anti-CD3) or antibody fragments thereof, major histocompatibility complex (MHC), etc.

In some options, the ICL is a molecular entity (e.g., protein, peptide, polypeptide, etc.) that interacts with an immune checkpoint receptor (ICR) on an effector cell (e.g., T cell, Jurkat, etc.) to modulate (e.g., inhibit, enhance, etc.) the immune response of the effector cell. In some options, the ICL is any suitable immune checkpoint ligand including but not limited to PD-L1, PD-L2, B7-H4, CD155, galectin-9, HVEM, etc.

In some options, the effector cell is a T cell selected from the group including, but not limited to, Jurkat cells, HuT-78, CEM, Molt-4, etc. In some options, the effector cell is artificial (e.g., engineered to contain, express, etc. one or more elements (e.g., a reporter construct, exogenous gene, etc.) not native to the parent (e.g., non-artificial) cell). In some options, the effector cell is an unengineered human or animal T cell.

In some options, the ICR is any suitable immune inhibitory receptor including, but not limited to, PD-1, CTLA-4, LAG-3, TIM-3, TIGIT, CD160, BTLA, IL-10 receptor, etc.

In some options, the TCR complex is an octomeric complex of variable TCR receptor α and β chains with three dimeric signaling modules CD3δ/ε, CD3γ/ε, and CD247 ζ/ζ or ζ/η.

In some options, described herein are methods of measuring the potency of a test blocking agent to inhibit the interaction of a immune checkpoint receptor and an immune checkpoint ligand, comprising: (a) co-culturing: (i) an effector cell displaying T Cell Receptor (TCR) and an immune checkpoint receptor (ICR) on its surface and a TCR activation pathway-dependent reporter; (ii) an artificial antigen presenting cell (aAPC) displaying an anti-cluster of differentiation 3 (CD3) antibody, or antibody fragment thereof, and an immune checkpoint ligand, wherein the ICR and ICL form an ICR/ICL complex upon interaction; and (iii) a test blockade agent; (b) incubating the mixture in (a) to allow signal induction; (c) detection of said signal from said reporter; and (d) comparing said signal from the cell co-culture system with and without the test blockade agent, wherein a gain of signal indicates inhibition of said ICR/ICL complex by said test blockade agent. In some options, the effector cell is a T cell including, but not limited to, Jurkat cells, HuT-78, CEM, Molt-4, etc. In some options, the reporter is a luciferase, a beta lactamase, CAT, SEAP, a fluorescent protein, etc. In some options, the ICR can be any immune inhibitory receptor including, but not limited to, PD-1, CTLA-4, LAG-3, TIM-3, TIGIT, CD160, BTLA, IL-10 receptor, etc., and the ICL can be any immune inhibitory ligand including, but not limited to, PD-L1, PD-L2, B7-H4, CD155, galectin-9, HVEM, etc. In some options, the blockade agent or test blockade agent is an antibody (or antibody fragment), protein, peptide, or small molecule or combination thereof.

In some options described herein are methods of measuring the potency of a test blocking agent to inhibit the interaction of a immune checkpoint receptor and an immune checkpoint ligand, comprising: (a) co-culturing: (i) an effector cell displaying T Cell Receptor (TCR) and an immune checkpoint receptor (ICR) on its surface, and a TCR activation pathway-dependent reporter (e.g., NFAT-promoter dependent reporter), and (ii) an artificial antigen presenting cell (aAPC) displaying a TCR activator (e.g., an anti-cluster of differentiation 3 (CD3) antibody or antibody fragment thereof) and an immune checkpoint ligand (ICL), wherein the ICR and ICL form an ICR/ICL complex upon interaction; (b) detecting a signal from said reporter; (c) adding said test blockade agent; (d) repeating detection of said signal from said reporter; and (e) comparing said signal from step (b) with said signal from step (d), wherein a gain of signal from step (b) to step (d) indicates inhibition of said ICR/ICL complex by said test blockade agent. In some options, the effector cell is a T cell including, but not limited to, Jurkat cells, HuT-78, CEM, Molt-4, etc. In some options, the reporter is a luciferase, a beta lactamase, CAT, SEAP, a fluorescent protein, gene expression (e.g., quantified by qPCR), etc. In some options, the ICR can be any immune inhibitory receptor including, but not limited to, PD-1, CTLA-4, LAG-3, TIM-3, TIGIT, CD160, BTLA, IL-10 receptor, etc., and the ICL can be any corresponding immune inhibitory ligand including, but not limited to, PD-L1, PD-L2, B7-H4, CD155, galectin-9, HVEM, etc. In some options, the blockade agent or test blockade agent is an antibody (or antibody fragment), protein, peptide or small molecule or combination thereof. In some options, the ICR is PD-1, and the ICL is PD-L1. In some options, the test blockade agent is an antibody or a small molecule.

In some options, described herein are methods of measuring the potency of a test blocking agent to inhibit the interaction of a immune checkpoint receptor and an immune checkpoint ligand, comprising: (a) co-culturing: (i) an effector cell displaying T Cell Receptor (TCR) and an immune checkpoint receptor (ICR) on its surface, and a TCR activation pathway-dependent reporter (e.g., NFAT-promoter dependent reporter), and (ii) an artificial antigen presenting cell (aAPC) displaying a TCR activator (e.g., an anti-cluster of differentiation 3 (CD3) antibody or antibody fragment thereof) and an immune checkpoint ligand (ICL), wherein the ICR and ICL form an ICR/ICL complex upon interaction; (b) detecting a signal from said reporter in the presence of a test blockade agent; and (c) comparing said signal from step (b) with a control (e.g., signal in the absence of said test blockade agent, a control signal, background, a zero control, a negative control, etc.), wherein a gain of signal relative to control indicates inhibition of said ICR/ICL complex by said test blockade agent. In some options, methods further provide a step of adding the test blockage agent.

In some options, described herein are methods comprising: (a) forming a system comprising: (i) an effector cell displaying on its surface an immune checkpoint receptor (ICR), and comprising a T Cell Receptor (TCR) and a TCR-pathway-dependent reporter, and (ii) an artificial antigen presenting cell (aAPC) displaying on its surface a TCR activator and an immune checkpoint ligand (ICL); wherein the ICR and ICL form an ICR/ICL complex upon interaction, and wherein formation of the ICR/ICL complex results in modulation of TCR activation by the TCR activator and/or modulation of one or more TCR-dependent pathways; and (b) detecting said TCR-pathway-dependent reporter or a signal from said reporter. In some options, methods further comprise adding to the system a blockade agent, wherein the blockade agent inhibits formation of the ICR/ICL complex or inhibits ICR/ICL-dependent modulation of TCR activation by the TCR activator and/or modulation of one or more TCR-dependent pathways. In some options, the TCR-pathway-dependent reporter or a signal from said reporter is detected: (1) before, (2) concurrent with, and/or (3) after addition of said blockade agent. In some options, systems further comprise a step of comparing signal from (1) before, (2) concurrent with, and/or (3) after addition of said blockade agent to determine the effect of the blockade agent.

In some options, described herein are methods comprising: (a) co-culturing:
(i) an effector cell displaying T Cell Receptor (TCR) and an immune checkpoint receptor (ICR) on its surface, and TCR-pathway-dependent reporter, and (ii) an artificial antigen presenting cell (aAPC) displaying a TCR activator and an immune checkpoint ligand (ICL), wherein the ICR and ICL form an ICR/ICL complex upon interaction; (b) adding said test blockade agent; (c) detecting said signal from said reporter; and (d) comparing said signal from said reporter with a control, wherein a gain of signal with respect to the control indicates inhibition of said ICR/ICL complex by said test blockade agent.

In some options, described herein are methods of identifying an ICR/ICL blockade agent, comprising: (a) co-culturing: (i) an effector cell displaying T Cell Receptor (TCR) and an immune checkpoint receptor (ICR) on its surface, and TCR-pathway-dependent reporter, and
(ii) an artificial antigen presenting cell (aAPC) displaying anti-cluster of differentiation 3 (CD3) antibody, or antibody fragment thereof, and an immune checkpoint ligand, wherein the ICR and ICL form an ICR/ICL complex upon interaction; (b) detecting a signal from said reporter; (c) adding said test blockade agent; (d) repeating detection of said signal from said reporter; and (e) comparing said signal from step (b) with said signal from step (d), wherein a gain of signal from step (b) to step (d) indicates inhibition of said ICR/ICL complex by said test blockade agent.

In some options, described herein are methods of identifying an ICR/ICL blockade agent, comprising: (a) contacting: (i) an effector cell displaying on its surface PD-1, and comprising: T cell Receptor (TCR) and a nuclear factor of activated T cells (NFAT) promoter-dependent luciferase reporter, and (ii) an artificial antigen presenting cell (aAPC) displaying anti-cluster-of-differentiation-3 (CD3) antibody, or antibody fragment thereof, and PD-L1; and (b) detecting a signal from said NFAT-promoter-dependent luciferase reporter. In some options, methods further comprise: (c) adding said test blockade agent; and (d) repeating detection of said signal from said reporter. In some options, methods further comprise: (e) comparing said signal from step (b) with said signal from step (d), wherein a gain of signal from step (b) to step (d) indicates inhibition of said ICR/ICL complex by said test blockade agent.

In some options, described herein are methods comprising administering an artificial antigen presenting cell (aAPC) displaying on its surface a T cell receptor (TCR) activator and an immune checkpoint ligand (ICL) to a system comprising a natural effector cell.

In some options, described herein are methods comprising administering an artificial antigen presenting cell (aAPC) displaying on its surface a T cell receptor (TCR) activator and an immune checkpoint ligand (ICL) to a system comprising an artificial effector cell.

### BRIEF DESCRIPTION OF THE DRAWINGS

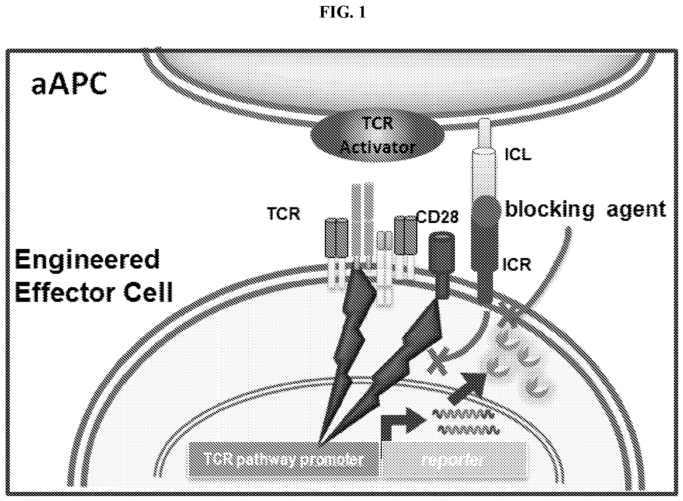

Figure 1 shows a schematic of a general system for assessing inhibitors of immune checkpoints. An aAPC displaying a T cell receptor (TCR) activator and immune checkpoint ligand (ICL) on its cell membrane is shown interacting with an engineered effector cell displaying an immune checkpoint receptor (ICR), CD28 and the TCR complex and comprising a TCR-pathway-promoter-dependent reporter construct. Upon co-culture of the aAPC and engineered effector cell, the TCR activator activates the TCR complex; however, the interaction of ICR and ICL inhibits activation of expression from the TCR pathway and little expression of the reporter occurs. In the presence of a blocking agent that inhibits the ICR/ICL interaction, the TCR pathway is activated, expression of the reporter form the TCR-pathway-promoter is enhanced, and reporter signal is elevated.

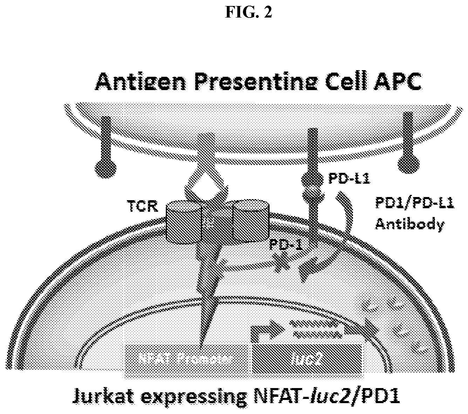

Figure 2 shows a schematic of an exemplary system for assessing inhibitors of immune checkpoints. An aAPC displaying PD-L1 and anti-CD3 antibody Fab domain on its cell membrane is shown interacting with a Jurkat T cell displaying PD-1, the TCR complex, and comprising NFAT-promoter-dependent luciferase reporter construct. Upon co-expression of the aAPC and Jurkat effector cell, anti-CD3 activates the TCR complex; however, the interaction of PD-1 and PD-L1 inhibits activation of expression from the NFAT promoter, and little expression of the luciferase occurs. In the presence of an inhibitor of the PD-1/PD-L1 interaction, the activated TCR complex activates expression of the luciferase reporter from the NFAT promoter, and luciferase signal is elevated.
Figure 3 shows a graph depicting the specificity of an exemplary PD-1/PD-L1 blockade assay.
Figure 4 shows graphs depicting concentration-dependent activation of reporter expression by an anti-PD-L1 antibody in an exemplary PD-1/PD-L1 blockade assay.
Figure 5 shows graphs depicting concentration-dependent activation of reporter expression by an anti-PD-1 antibody in an exemplary PD-1/PD-L1 blockade assay.
Figure 6 shows a schematic of system analogous to the one depicted in Figure 2, but the APC does not express TCR activator.
Figure 7 shows graphs depicting concentration-dependent activation of reporter expression by an anti-PD-L1 antibody in a PD-1/PD-L1 blockade assay with aAPCs lacking engineered membrane-bound anti-CD3.
Figure 8 show graphs depicting the absence of PD-1/PD-L1-dependent inhibition of T- cell activation by anti-CD3/PD-L1-Fc beads.
Figures 9A-9B show flow cytometry results confirming the attachment of PD-L1 to beads.
Figure 10 shows a graph demonstrating that anti-CD3:PD-L1 beads (1:9 ratio) failed to induce inhibition of T cell activation, and therefore that anti-PD-1/PD-L1 antibody failed to induce T cell activation.
Figure 11 shows a graph demonstrating that extended length anti-PD-L1-Fc on anti-CD3:PD-L1 beads failed to induce inhibition of T cell activation, and therefore that anti-PD-1/PD-L1 antibody failed to induce T cell activation.
Figure 12 shows a contemplated mechanistic explanation for the failure of anti-CD3:PD-L1 beads to perform like anti-CD3:PD-L1 aAPCs in exemplary blockade assays.
Figure 13 shows a schematic of an exemplary system for assessing inhibitors of immune checkpoint receptor PD-1 and its ligand, PD-L2.
Figures14A-14B show graphs depicting concentration-dependent activation of reporter expression by an anti-PD-1 antibody (b) or an anti-PD-L2 (a) antibody in an exemplary PD-1/PD-L2 blockade assay.
Figure 15 shows a schematic of an exemplary system for assessing inhibitors of immune checkpoint receptor CTLA-4 and its ligand, CD80/CD86.
Figure 16 shows a graph depicting concentration-dependent activation of reporter expression by an anti-CTLA-4 antibody in an exemplary CTLA-4 blockade assay.
Figure 17 shows a schematic of an exemplary system for assessing inhibitors of immune checkpoint receptor TIGIT and its ligand, CD155.
Figure 18 shows a graph depicting concentration-dependent activation of reporter expression by an anti-TIGIT antibody in an exemplary TIGIT/CD155 blockade assay using a TIGIT Effector Cells in Jurkat-T cells.
Figure 19 shows a schematic of an exemplary system for assessing inhibitors of immune checkpoint receptor TIGIT and its ligand, CD155.
Figure 20 shows a graph depicting concentration-dependent activation of reporter expression by an anti-TIGIT antibody in an exemplary TIGIT/CD155 blockade assay using a TIGIT Effector Cells in CEM T cells.
Figure 21 shows a schematic of an exemplary system for assessing inhibitors of immune checkpoint receptor PD-1 and its ligand PD-L1.
Figure 22 shows a graph depicting concentration-dependent IL-2 production by an anti-PD-1 antibody in an exemplary PD-1/PD-L1 blockade assay.
Figure 23 shows a schematic of an exemplary system for assessing inhibitors of immune checkpoint receptor LAG-3 and its ligand, MHC II.
Figure 24 shows graphs depicting concentration-dependent activation of reporter expression by an anti-LAG-3 antibody in an exemplary LAG-3 blockade assay.

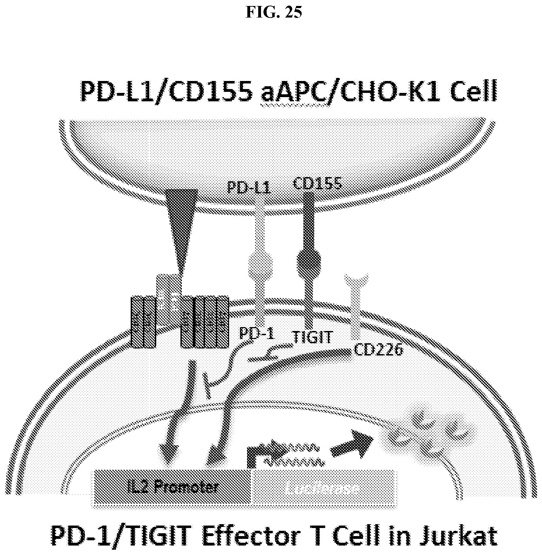

Figure 25 shows a schematic of an exemplary system for assessing inhibitors of immune checkpoint receptors TIGIT and PD-1 and their ligands, CD155 and PD-L1, respectively. An aAPC displaying PD-L1 and CD155 and anti-CD3 antibody Fab domain on its cell membrane is shown interacting with a Jurkat T cell displaying PD-1, co-stimulatory receptor CD226, TIGIT, the TCR complex, and comprising IL2-promoter-dependent luciferase reporter construct. Upon the interaction of the aAPC and Jurkat effector cell, anti-CD3 Ab activates the TCR complex and CD155 co-stimulates CD226. These lead to activation of IL-2 promoter driving luciferase expression; however, the interaction of PD-1 with PD-L1 leads to reduced TCR pathway activation and reduced IL-2 promoter-dependent reporter expression. In addition, CD155 has higher binding affinity with TIGIT than with CD226. Therefore, TIGIT inhibits interaction of CD155 with CD226 and leads to reduced reporter expression. In the presence of an inhibitor of the PD-1/PD-L1 and/or TIGIT/CD155 interaction, the expression of the luciferase reporter from IL-2 promoter is enhanced, and the luciferase signal is elevated.
Figure 26a-b shows graphs depicting concentration-dependent activation of reporter expression by (a) an anti-PD-1 antibody and (b) an anti-TIGIT antibody in an exemplary PD-1/TIGIT blockade assay using a PD-1/TIGIT Effector Cells in Jurkat-T cells and PD-L1/CD155 aAPC/CHO-K1 cells. Figure 26c shows the effects of a combination of anti-PD-1 and anti-TIGIT antibodies compared with either antibody alone.

### DEFINITIONS

As used herein, the term "immune checkpoint receptor" ("ICR") refers to a surface receptor protein on an immune cell (e.g., T cell, Jurkat cells, etc.) that modulates the immune activity of the cell when bound to its ligand. Of particular interest herein are "inhibitory immune checkpoint receptors" which inhibit cellular immune activity upon ligand binding to the receptor. Examples of "inhibitory immune checkpoint receptors" include, but are not limited to, PD-1, CTLA-4, LAG-3, TIM-3, CD160, TIGIT, IL-10 receptor, and BTLA.

As used herein, the term "immune checkpoint ligand" ("ICL") refers to a ligand of an immune checkpoint receptor. "Immune checkpoint ligands" are commonly surface-displayed proteins on antigen presenting cells (APCs). Through an interaction with an immune-cell-displayed immune checkpoint receptor, an "immune checkpoint ligand" modulates the immune response of the immune cell (e.g., T cell) to the antigen presenting cell. Examples of "immune checkpoint ligands" that bind inhibitory immune checkpoint receptors include, but are not limited to, PD-L1, PD-L2, B7-H4, CD155, galectin-9, HVEM, etc.

As used herein, the terms "immune checkpoint," "checkpoint pathway," and "immune checkpoint pathway" refer to a pathway by which the binding of an immune checkpoint ligand to an immune checkpoint receptor modulates the amplitude and quality of the activation of immune cells (e.g., T cells, Jurkat cells, HuT-78, CEM, Molt-4, etc.).

As used herein, the term "immune checkpoint blockade" refers to the inhibition of an immune checkpoint pathway by the administration or expression of a "blockade agent." Typically, the "blockade agent" prevents the interaction of the immune checkpoint receptor and ligand, thereby inhibiting the checkpoint pathway. A blockade agent may be a small molecule, peptide, antibody or fragment thereof, etc. that binds to an immune checkpoint ligand or immune checkpoint receptor and inhibits the formation of the ICR/ICL complex. A blockade agent may also function by preventing signaling by the ICR/ICL complex.

As used herein, the term "artificial," as in "artificial antigen presenting cell" or "artificial effecter cell," refers to an entity (e.g., cell, construct, etc.) that does not occur in nature. For example, a cell engineered to express an exogenous gene (e.g., reporter construct) that is not intrinsic to the parent (e.g., unengineered cell) is "artificial."

As used herein, the term "antibody" refers to a protein having one or more polypeptides substantially encoded by immunoglobulin genes or fragments of immunoglobulin genes. The recognized immunoglobulin genes include the kappa, lambda, alpha, gamma, delta, epsilon and mu constant region genes, as well as the myriad of immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. The basic immunoglobulin (antibody) structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

The term "antibody" is used herein in the broadest sense and specifically covers human, non-human (e.g. murine), and humanized monoclonal antibodies (including full length monoclonal antibodies), polyclonal antibodies, multi-specific antibodies (e.g., bispecific antibodies), single-chain antibodies, and antibody fragments so long as they exhibit the desired biological activity. Antibodies may exist as intact immunoglobulins, or as modifications in a variety of forms including, for example, FabFc₂, Fab, Fv, Fd, (FabN)₂, an Fv fragment containing only the light and heavy chain variable regions, a Fab or (Fab)N₂ fragment containing the variable regions and parts of the constant regions, a single-chain antibody, e.g., scFv, CDR-grafted antibodies and the like. The heavy and light chain of an Fv may be derived from the same antibody or different antibodies thereby producing a chimeric Fv region. The antibody may be of animal (especially mouse or rat) or human origin or may be chimeric or humanized. As used herein the term "antibody" includes these various forms.

The term "reporter" is used herein in the broadest sense to describe a molecular entity, a characteristic and/or property of which (e.g., concentration, amount, expression, activity, localization, interactions (e.g., protein-protein interactions), etc.) can be detected and correlated with a characteristic and/or property of a system containing the reporter (e.g., cell, cell lysate, in vitro system, organism, in vivo system, etc.). A "reporter" may be an intrinsic (e.g., endogenous) element of the system that exhibits one or more detectable and correlatable properties, or an artificial (e.g., exogenous) element engineered or introduced into the system, that exhibits a detectable characteristic linked to process (e.g., gene expression) or component within the system. Suitable reporters include, but are not limited to: intrinsic genes or proteins (e.g., expression, concentration, activity, or protein-protein interactions of which may be correlated to system (e.g., cellular processes)), exogenous genes or proteins (e.g., expression, concentration, activity, or protein-protein interactions of which may be correlated to system (e.g., cellular processes)), luciferases, beta lactamases, CAT, SEAP, fluorescent proteins, etc.

### DETAILED DESCRIPTION

Described herein are compositions, systems, and methods for assessing modulators of immune checkpoints. In particular, artificial antigen presenting cells (aAPCs) and immune effector cells are provided to assess the potency of test agents (e.g., antibodies) to inhibit immune checkpoints.

In some options, described herein are compositions comprising an artificial antigen presenting cell (aAPC) comprising (e.g., displaying on its surface): (1) A TCR activator and (2) an immune checkpoint ligand (ICL).

An antigen-presenting cell (APC), or accessory cell, is a cell that processes and presents foreign antigens complexed with major histocompatibility complexes (MHCs) on its surface, in a process is known as antigen presentation. T cells may recognize these complexes using their T cell receptors (TCRs) and/or T cell receptor complexes. Artificial antigen presenting cells (aAPCs) are typically derived from primary or transformed human or xenogeneic cells that are engineered (e.g., using retroviral or lentiviral transduction) to introduce molecules that provide the co-stimulatory and adhesion properties required for immune synapse formation. This strategy allows stringent control of the delivery of the many positive and negative signals to T cells during the interaction with the aAPC (See, e.g., Turtle et al. (Cancer J. 2010 Jul-Aug; 16(4):374-81) for review of aAPCs in immunotherapy).

In some options, aAPCs are provided that express and/or display on their surface an activator of TCR. In some options, interaction of a TCR activator with TCR results in activation of TCR-dependent pathways and enhanced gene expression from promoters downstream of TCR (e.g., NFAT-dependent expression). In some options, aAPCs are provided that express anti-CD3 antibody (e.g., full antibody, Fab domain, etc.). In some options, aAPCs are provided that display anti-CD3 antibody (e.g., full antibody, Fab domain, etc.) on their surface. It has been demonstrated that resting T-lymphocytes are activated by anti-CD3 antibodies (Tsoukas et al, J Immunol. 1985 Sep; 135(3): 171).

In some options, anti-CD3-expressing aAPCs activate T cells (e.g., Jurkat cells) through the binding of anti-CD3 to surface-displayed CD3 within the TCR complex of the T cells (e.g., Jurkat cells). In some options, anti-CD3 binding to the TCR complex results in a series of downstream biochemical events mediated by associated enzymes, co-receptors, specialized adaptor molecules, and activated or released transcription factors. For example, anti-CD3 binding to the TCR results in activation of NFAT proteins (e.g., NFATc1, NFATc2, NFATc3, NFATc4, NFAT5) and increased gene expression from NFAT promoters (e.g., promoters that bind NFAT resulting in enhanced transcription). In some options, expression of an NFAT-promoter-linked reporter (e.g., luciferase) provides a quantitative measure of T cell activation and immune checkpoint blockade. In some options, other downstream events triggered by the binding of anti-CD3 to the TCR provide suitable markers of T cell activation (or can be manipulated to provide markers of as much). Other examples of T cell activators (e.g., TCR activators) include superantigens, anti-TCR antibodies, anti-CD2 antibodies, anti-CD4 antibodies, PHA, MHC and cognate peptides, and Con A, any of which may be expressed in aAPCs and/or surface displayed thereon to activate effector cells.

In some options, aAPCs are provided that express an ICL (e.g., for use in systems comprising effector cells displaying ICR). In some options, aAPCs are provided that display ICL on their surface including, but not limited to, PD-L1, PD-L2, B7-H4, CD155, galectin-9, HVEM, etc. In some options, aAPCs express and/or display a peptide/polypeptide with at least 50% sequence identity (e.g., >50%, >60%, >70%, >80%, >90%, >95%, or more) with all or a portion of ICL, and retain at least 50% (e.g., >50%, >60%, >70%, >80%, >90%, >95%, or more) of the affinity of ICL for ICR. In some options, aAPCs are provided that display or express a peptide/polypeptide that has at least 50% (e.g., >50%, >60%, >70%, >80%, >90%, >95%, or more) of the affinity of ICL for ICR.

In some options, aAPCs are provided that express PD-L1 (e.g., for use in systems comprising effector cells displaying PD-1). In some options, aAPCs are provided that display PD-L1 on their surface. In some options, aAPCs express and/or display a peptide/polypeptide with at least 50% sequence identity (e.g., >50%, >60%, >70%, >80%, >90%, >95%, or more) with all or a portion of ICL and retain at least 50% (e.g., >50%, >60%, >70%, >80%, >90%, >95%, or more) of the affinity of PD-L1. In some options, aAPCs are provided that display or express a peptide/polypeptide that has at least 50% (e.g., >50%, >60%, >70%, >80%, >90%, >95%, or more) of the affinity of PD-L1 for PD-1.

In some options, described herein are systems comprising: (a) an artificial antigen presenting cell comprising (e.g., displaying on its surface): (i) a T cell receptor (TCR) activator, and (ii) an immune checkpoint ligand (ICL) (e.g., any of the aAPCs described in above); and (b) an artificial effector cell comprising an immune checkpoint receptor (ICR) (e.g., displayed on its surface) and comprising: (i) a TCR complex and (ii) a reporter (e.g., reporter of one or more of: (A) ICR/ICL complex formation, (B) TCR activation, and/or (C) the TCR signaling pathway TCR activation pathway-dependent reporter).

In certain options, systems are provided comprising an aAPC (e.g., as described in Section I above or elsewhere herein) and an effector cell (e.g., artificial effector cell). An effector cell is a lymphocyte (e.g., T cell, Jurkat cell, etc.) that has been induced to differentiate into a form capable of mounting a specific immune response. In some options, effector cells are provided that express and/or display surface proteins and/or complexes of surface proteins useful in the blockade assays described herein (e.g., an antigen-responsive element (e.g., TCR), ICR (e.g., PD-1), etc.). In some options, an effector cell useful in the options described herein has been engineered to express a reporter of effector cell activation. In some options, one or more pathways involved in the activation of T cells activate the reporter and/or expression of the reporter, thereby detectably signaling blockade of the immune checkpoint and T cell activation.

In some options, an effector cell displays numerous markers indicative of a T cell (e.g., human T cell, Jurkat cell, etc.). For example, an effector cell may display one or more of: the T cell receptor complex and/or components thereof (e.g., CD3, TCRα, TCRβ, TCRγ, TCRδ, etc.), CD4, CD8, CD28, CTLA-4, CD40L, CD2, LFA-1, etc.

Activation of T cells occurs through the simultaneous engagement of the T cell receptor and a co-stimulatory molecule on the T cell by the major histocompatibility complex (MHCII) peptide and co-stimulatory molecules on an antigen presenting cell (APC). Once properly engaged by the APC, downstream signaling pathways are initiated to activate the T cell. For example, co-stimulatory molecules engage the phosphoinositide 3-kinase (PI3K) pathway, generating phosphatidylinositol (3,4,5)-trisphosphate (PIP3) at the plasma membrane and recruiting PH-domain-containing signaling molecules (e.g., pyruvate dehydrogenase lipoamide kinase isozyme 1 (PDK1)) that are essential for the activation of PKCθ, and eventual IL-2 production. A non-limiting set of downstream effects of the activation include: phosphorylation of CD28, LAT and SLP-76, which allows the aggregation of signaling complexes around these proteins; recruitment of SLP-76 to the membrane, where it can then bring in PLC-γ, VAV1, Itk and potentially PI3K; activation of transcription factors, such as NF-κB and AP-1; activation of calcium receptors on the endoplasmic reticulum (ER); release of calcium from the ER into the cytosol which causes STIM1 clustering on the ER membrane and leads to activation of cell membrane CRAC channels that allow additional calcium to flow into the cytosol from the extracellular space; binding of calmodulin by aggregated cytosolic calcium, which then activates calcineurin; calcineurin activation of nuclear factor of activated T cells (NFAT), translocation of NFAT to the nucleus; NFAT activation of the transcription of a pleiotropic set of genes, most notably, IL-2, interferons, tumor necrosis factor, IL-17, IL-10, IL-4, IL-13, IL-23, IL-1, transforming growth factor beta, IL-8 and other chemokines, and numerous other pathways. In some options, any of these downstream effects may find use as a marker of blockade of an immune checkpoint (e.g., by liking to downstream effect to a detectable reporter).

In some options, systems and methods are provided for assessing the potency of blockade agents (e.g., antibodies) in inhibiting immune checkpoint pathways. Exemplary systems comprise: (1) an artificial effector cell (e.g., T cell (e.g., Jurkat cell, etc.), etc.) displaying an antigen-responsive element (e.g., T Cell Receptor (TCR) complex) and an immune checkpoint receptor (ICR) on its surface (e.g., PD-1) and comprising a reporter; (2) an artificial antigen presenting cell (aAPC) displaying on its surface an activator of the antigen responsive element of the effector cell (e.g., anti-CD3 antibody or a fragment thereof) and an immune checkpoint ligand (ICL) corresponding to the ICR of the effector cell (e.g., PD-L1); and (3) a test blockade agent (e.g., antibody to the ICL or ICR). In some options, in the absence of the blockade agent (or in the event that the test blockade agent is ineffective), the ICR and ICL interact and modulate (e.g., inhibit or enhance) the signal from the reporter construct. However, in the presence of a potent blockade agent, the interaction of the ICR and ICL is inhibited and modulation the reporter signal by the ICR/ICL is inhibited.

In some options, systems and methods are provided for assessing the potency of blockade agents (e.g., antibodies) in blocking inhibitory immune checkpoint pathways. Exemplary systems comprise: (1) an artificial effector cell (e.g., engineered T cell (e.g., Jurkat cell, etc.), etc.) displaying an antigen-responsive element (e.g., T Cell Receptor (TCR) complex) and an immune checkpoint receptor (ICR) on its surface and comprising a reporter construct, the expression of which is dependent upon activation of the antigen-responsive element; (2) an artificial antigen presenting cell (aAPC) displaying on its surface an activator of the antigen responsive element of the effector cell and an immune checkpoint ligand (ICL) corresponding to the ICR of the effector cell; and (3) a test blockade agent (e.g., antibody to the ICL or ICR). In some options, activation of the antigen responsive element by interaction with the activator of the antigen responsive element results in a signal (or absence of signal) from the reporter. In some options, in the absence of the blockade agent (or in the event that the test blockade agent is ineffective), the ICR and ICL interact and modulate the signal from the reporter. In the presence of a potent blockade agent, the interaction of the ICR and ICL is inhibited, modulation of the reporter signal by the ICR/ICL is blocked, and the reporter signal tends toward the signal in the absence of an ICR/ICL. In some options, when effector cells and aAPCs are co-cultured, a first signal level is produced from the reporter (e.g., because the ICR/ICL complex modulates reporter signal); however, addition of a potent blockade agent results in a second signal level from the reporter (e.g., because formation of the ICR/ICL is inhibited, and therefore modulation of reporter signal by the ICR/ICL complex is reduced).

In some options, systems and methods are provided for assessing the potency of blockade agents (e.g. antibodies) in inhibiting immune checkpoint pathways. Exemplary systems comprise: (1) an effector cell (e.g., T cell (e.g., Jurkat cell, etc.), etc.) displaying T Cell Receptor (TCR) complex and an immune checkpoint receptor (ICR) on its surface and expressing a NFAT-promoter-dependent reporter (e.g., luciferase); (2) an artificial antigen presenting cell (aAPC) displaying anti-CD3 antibody (or a fragment thereof) and an immune checkpoint ligand (ICL) corresponding to the ICR of the effector cell; and (3) a test blockade agent (e.g., antibody to the ICL or ICR). In some options, in the absence of the blockade agent (or in the event that the test blockade agent is ineffective), the ICR and ICL interact and inhibit activation of the NFAT promoter by the CD3-antibody-bound TCR complex, thereby inhibiting expression of the reporter. However, in the presence of a potent blockade agent, the interaction of the ICR and ICL is inhibited, the NFAT promoter is activated, and the reporter is expressed. In some options, when effector cells and aAPCs are co-cultured, little or no signal is produced from the reporter (e.g., because the ICR/ICL complex inhibits reporter expression); however, addition of a potent blockade agent results in increased expression from the NFAT promoter and a gain of signal from the reporter.

In some options, systems and methods are provided for assessing the potency of blockade agents (e.g. antibodies) in inhibiting the PD-1/PD-L1 immune checkpoint pathway. Exemplary systems comprise: (1) a Jurkat cell displaying T Cell Receptor (TCR) complex and PD-1 on its surface and expressing an NFAT-promoter-induced luciferase reporter (e.g., luc2); (2) an artificial antigen presenting cell (aAPC) displaying anti-CD3 antibody and PD-L1 on its surface; and (3) a test blockade agent (e.g., antibody to PD-1 or PD-L1). In some options, in the absence of the blockade agent (or in the event that the test blockade agent is ineffective), the PD-1 and PD-L1 interact and inhibit activation of the NFAT promoter by the anti-CD3-antibody-bound TCR complex; therefore, luciferase expression is inhibited, and the reporter signal is diminished. In the presence of a potent blockade agent, the interaction of the PD-1, and PD-L1 is inhibited, the NFAT promoter is activated, luciferase expression is enhanced, and the reporter signal increases. In some options, when Jurkat effector cells and aAPCs are co-cultured, little or no luciferase is expressed, and little or no signal is generated (e.g., because the PD-1/PD-L1 complex inhibits reporter expression); however, addition of a potent blockade agent results in increased expression of luciferase and a gain of signal from the system.

In some options, systems, and methods are provided for assessing the potency of blockade agents (e.g. antibodies) in inhibiting the PD-1/PD-L2 immune checkpoint pathway. Exemplary systems comprise: (1) a Jurkat cell displaying T Cell Receptor (TCR) complex and PD-1 on its surface and expressing an NFAT-promoter-induced luciferase reporter (e.g., luc2); (2) an artificial antigen presenting cell (aAPC) displaying anti-CD3 antibody and PD-L2 on its surface; and (3) a test blockade agent (e.g., antibody to PD-1 or PD-L2). In some options, in the absence of the blockade agent (or in the event that the test blockade agent is ineffective), the PD-1 and PD-L2 interact and inhibit activation of the NFAT promoter by the anti-CD3-antibody-bound TCR complex; therefore, luciferase expression is inhibited, and the reporter signal is diminished. In the presence of a potent blockade agent, the interaction of the PD-1, and PD-L2 is inhibited, the NFAT promoter is activated, luciferase expression is enhanced, and the reporter signal increases. In some options, when Jurkat effector cells and aAPCs are co-cultured, little or no luciferase is expressed, and little or no signal is generated (e.g., because the PD-1/PD-L2 complex inhibits reporter expression); however, addition of a potent blockade agent results in increased expression of luciferase and a gain of signal from the system.

In some options, systems, and methods are provided for assessing the potency of blockade agents (e.g. antibodies) in inhibiting an immune checkpoint pathway dependent upon the interaction of CD80/CD86 and CTLA-4. Exemplary systems comprise: (1) a Jurkat cell displaying co-stimulatory receptor CD28, CTLA-4, the TCR complex, and comprising IL-2-promoter-dependent luciferase reporter construct; (2) an artificial antigen presenting cell (aAPC) displaying CD80/CD86 and anti-CD3 antibody Fab domain on its cell membrane; and (3) a test blockade agent (e.g., antibody to CTLA-4 or CD80/CD86). Upon the interaction of the aAPC and Jurkat effector cell, anti-CD3 Ab activates the TCR complex and CD80/CD86 co-stimulates CD28. This leads to activation of the IL-2 promoter, driving luciferase expression; however, CTLA-4 has higher binding affinity with CD80/CD86 than its binding affinity with CD28. It inhibits interaction of CD80/CD86 with CD28, and luciferase expression from the IL-2 promoter. In the presence of an inhibitor of CTLA-4 with CD80/CD86 interaction, the activated CD28 activates expression of the luciferase reporter from IL-2 promoter, and luciferase signal is elevated.

In some options, systems, and methods are provided for assessing the potency of blockade agents (e.g. antibodies) in inhibiting the TIGIT/CD155 immune checkpoint pathway. Exemplary systems comprise: (1) a Jurkat T cell displaying co-stimulatory receptor CD226, TIGIT, the TCR complex, and comprising IL-2-promoter-dependent luciferase reporter construct; (2) an artificial antigen presenting cell (aAPC) displaying CD155 and anti-CD3 antibody Fab domain on its cell membrane; and (3) a test blockade agent (e.g., antibody to TIGIT or CD155). Upon the interaction of the aAPC and Jurkat effector cell, anti-CD3 Ab activates the TCR complex and CD155 co-stimulates CD226. These lead to activation of IL-2 promoter driving luciferase expression; however, CD155 has higher binding affinity with TIGIT than with CD226. Therefore, TIGIT inhibits interaction of CD155 with CD226 and the activation of luciferase expression from the IL-2 promoter. In the presence of an inhibitor of the TIGIT/CD155 interaction, the activated CD226 activates expression of the luciferase reporter from IL-2 promoter, and luciferase signal is elevated.

In some options, systems and methods are provided for assessing the potency of blockade agents (e.g. antibodies) in inhibiting the TIGIT/CD155 immune checkpoint pathway. Exemplary systems comprise: (1) a CEM T cell displaying co-stimulatory receptor CD226, TIGIT, the TCR complex, and comprising IL-2-promoter-dependent luciferase reporter construct (e.g., artificial CEM effector cell); (2) an artificial antigen presenting cell (aAPC) displaying CD155 and anti-CD3 antibody Fab domain on its cell membrane; and (3) a test blockade agent (e.g., antibody to TIGIT or CD155). Upon the interaction of the aAPC and CEM effector cell, anti-CD3 Ab activates the TCR complex and CD155 co-stimulates CD226. Both lead to activation of IL-2 promoter driving luciferase expression; however, CD155 has higher binding affinity with TIGIT than with CD226. Therefore, TIGIT inhibits interaction of CD155 with CD226 and the activation of luciferase expression from the IL-2 promoter. In the presence of an inhibitor of the TIGIT/CD155 interaction, the activated CD226 activates expression of the luciferase reporter from IL-2 promoter, and luciferase signal is elevated.

In some options, systems, and methods are provided for assessing the potency of blockade agents (e.g. antibodies) in inhibiting the PD-1/PD-L1 immune checkpoint pathway. Exemplary systems comprise: (1) a Jurkat T cell displaying PD-1, the TCR complex, and endogenous IL-2 gene driven by native IL-2 promoter; (2) an artificial antigen presenting cell (aAPC) displaying PD-L1 and anti-CD3 antibody Fab domain on its cell membrane; and (3) a test blockade agent (e.g., antibody to PD-1 or PD-L1). Upon the interaction of the aAPC and Jurkat effector cell, anti-CD3 Ab activates the TCR complex, activates IL-2 promoter, and increases IL-2 production; however, the interaction of PD-1 and PD-L1 inhibits activation of IL-2 expression from IL-2 promoter, and little production of IL-2 proteins occurs. In the presence of an inhibitor of the PD-1/PD-L1 interaction, the activated TCR complex activates IL-2 expression from IL-2 promoter, and IL-2 production in the media is elevated.

In some options, systems and methods are provided for assessing the potency of blockade agents (e.g. antibodies) in inhibiting the LAG-3/MHC II immune checkpoint pathway. Exemplary systems comprise: (1) a Jurkat T cell displaying LAG-3, the TCR complex, and comprising NFAT-promoter-dependent luciferase reporter construct; (2) an artificial antigen presenting cell (aAPC) in a Raji B cell displaying MHC II on its cell membrane; and (3) a test blockade agent (e.g., antibody to LAG-3 or MHC II). Upon the interaction of the APC and Jurkat effector cell, superantigen presented by MHC II activates the TCR complex and activation of NFAT promoter driving luciferase expression; however, LAG-3 binding with MHC II inhibits TCR activation. This leads to inactivation of luciferase expression from the NFAT promoter. In the presence of an inhibitor of LAG-3/MHC II interaction, the activated TCR activates expression of the luciferase reporter from NFAT promoter, and the luciferase signal is elevated.

In some options, systems comprise any suitable combination of the aAPCs, effector cells, ICLs, ICRs, promoters, reporters, cell types, etc. described herein.

In some options, systems and methods described herein make use of the activation of transcription from the TCR activation pathway, downstream from proper engagement of the TCR by an APC (e.g., anti-CD3 antibody binding to the TCR complex). In some options, effector cells are provided (e.g., engineered) with a reporter construct that signals activation of the cells. In some options, a construct is provided with a reporter (e.g., luciferase) gene under the control of a TCR activation pathway. Upon activation of the T cell activation pathways, increased expression of the reporter (e.g., luciferase) occurs and cell activation can be detected and/or quantified based on the signal from the reporter. As noted above, it has been experimentally demonstrated that resting T-lymphocytes can be activated by anti-CD3 antibodies (Tsoukas et al, J Immunol. 1985 Sep;135(3):1719-23).

Options described herein are not limited to activation by anti-CD3 In some options, other T cell stimulatory signals (e.g., displayed on aAPCs) are used to activate T cells, which is detected by a TCR activation pathway-dependent reporter. When effector cells comprising TCR activation pathway-dependent reporters are contacted by aAPCs displaying anti-CD3 antibodies (or fragments thereof), the T cell activation pathways are activated, and the reporter signal increases. However, upon formation of an inhibitory ICR/ICL complex (e.g., between an ICR on the effector cell and an ICL on the aAPC), T cell activation by the properly engaged TCR is inhibited, and transcription of the reporter from the TCR activation pathway is reduced. Blockade of the ICR/ICL complex by a blockade agent, restores TCR activation of T cell, and transcription of the reporter from the TCR activation pathway is increased.

In some options, a TCR activation pathway-dependent reporter construct comprises: (1) a TCR activation pathway response element or promoter, and (2) a nucleic acid encoding a detectable reporter peptide, polypeptide or protein. In some options, effector cells (e.g., Jurkat cells) are stably transfected with a TCR activation pathway-dependent reporter construct. Transfection is performed by methods well understood in the art; suitable methods include chemical-based transfection, non-chemical transfection, particle-based transfection, viral-based transfection, electroporation, and gene editing technology to monitor endogenous promoter activity or other known transfection methods.

An NFAT promoter is a nucleic acid sequence that contains one or more NFAT response elements. When bound by an NFAT transcription factor (e.g., NFATc1, NFATc2, NFATc3, NFATc4, or NFAT5), transcription of associated nucleic acid sequences (e.g., downstream genes) is enhanced. One suitable NFAT response element is:
GGAGGAAAAACTGTTTCATACAGAAGGCGT (SEQ ID NO: 1)

In some options, an NFAT promoter comprises repeats of SEQ ID NO: 1. In some options, an NFAT promoter comprises one or more NFAT response elements having at least 50% (e.g., >50%, >60%, >70%, >80%, >90%, >95%, and ranges therein) sequence identity to SEQ ID NO: 1, wherein the NFAT response elements bind to an NFAT family transcription factor to enhance transcription of associate (e.g., downstream) genes.

In some options, the TCR activation pathway response element or promoter is an IL-2 promoter sequence, an NFkappaB response element that binds to an NFkappaB family transcription factor to enhance transcription of associated (e.g., downstream) genes, an AP1 response elements bind to an AP1 family transcription factor to enhance transcription of associated (e.g., downstream) genes, or any combination of the above response elements.

In some options, the reporter of the TCR activation pathway-dependent reporter construct is any peptide, polypeptide, or protein that produces a detectable signal including, but not limited to, a luciferase, a beta lactamase, CAT, SEAP, a fluorescent protein, etc. In some options, the reporter is a fluorescent or bioluminescent reporter. In certain options, a bioluminescent reporter is a luciferase. In some options, a luciferase is selected from those found in *Omphalotus olearius,* fireflies (e.g., *Photinini), Renilla reniformis,* mutants thereof, portions thereof, variants thereof, and any other luciferase enzymes suitable for the systems and methods described herein. Options described herein are not limited by the potential identity of the reporter.

In some options, effector cells are provided that express PD-1 (e.g., for use in systems comprising aAPCs displaying PD-L1). In some options, effector cells are provided that display PD-1 on their surface. In some options, effector cells express and/or display a peptide/polypeptide with at least 50% sequence identity (e.g., >50%, >60%, >70%, >80%, >90%, >95%, or more) with all or a portion of PD-1, and retain at least 50% (e.g., >50%, >60%, >70%, >80%, >90%, >95%, or more) of the affinity of PD-1 for PD-L1. In some options, effector cells are provided that display or express a peptide/polypeptide that has at least 50% (e.g., >50%, >60%, >70%, >80%, >90%, >95%, or more) of the affinity of PD-1 for PD-L1, and at least 50% e.g., >50%, >60%, >70%, >80%, >90%, >95%, or more) of the capacity of PD-1 to inhibit activation of T cells upon binding to PD-L1.

In some options, effector cells are provided that express CD28, CTLA-4, CD226, TIGIT, etc. In some options, effector cells express and/or display a peptide/polypeptide with at least 50% sequence identity (e.g., >50%, >60%, >70%, >80%, >90%, >95%, or more) with all or a portion of CD28, CTLA-4, CD226, TIGIT, etc., and retain at least 50% (e.g., >50%, >60%, >70%, >80%, >90%, >95%, or more) of the affinity of CD28, CTLA-4, CD226, TIGIT, etc.) to ligands thereof (e.g., CD80/CD86, CD155, etc.). In some options, effector cells are provided that display or express a peptide/polypeptide that has at least 50% (e.g., >50%, >60%, >70%, >80%, >90%, >95%, or more) of the affinity of a receptor (e.g., CD28, CTLA-4, CD226, TIGIT, etc.) for a ligand thereof (e.g., CD80/CD86, CD155, etc.), and at least 50% (e.g., >50%, >60%, >70%, >80%, >90%, >95%, or more) of the capacity of the receptor to effect activation of T cells upon binding to the appropriate ligand.

In some options, effector cells are provided that express and/or display one or more ICRs other than PD-1 (e.g., for use in systems comprising aAPCs displaying ICLs other than PD-L1). For example, in some options, effector cells are provided that express and/or display one or more of CTLA-4, BTLA, etc. Options described herein are not limited to the listed ICRs. Any ICR capable of interacting with an ICL to inhibit the activity of an effector cell (e.g., T cell) may find use in the options described herein.

In some options, systems described herein comprise aAPCs and effector cells, wherein the aAPCs have the potential to activate the effector cells (e.g., via anti-CD3 of the aAPC binding to the TCR complex of the effector cell), thereby eliciting the expression of a detectable reporter (e.g., NFAT-promoter-dependent reporter); however, the interaction of an ICL (PD-L1) on the aAPC and an ICR (PD-1) on the effector cells inhibits effector-cell activation and reporter expression. In some options, exposing the system to a blockade agent inhibits the ICR/ICL interaction, enhancing effector cell activation, and increasing reporter expression. In some options, the potency of the blockade agent is directly proportional to reporter expression.

In certain options, it is the interaction of PD-1 and PD-L1 that limits the activity of the effector cell and reporter expression. Therefore, blockade of the PD-1/PD-L1 interaction activates effector cells and increases reporter expression and reporter signal. PD-1/PD-L1 blockade can be accomplished by a variety of mechanisms including antibodies that bind PD-1 or PD-L1. Examples of PD-1 and PD-L1 blockers are described in US Patent Nos. 7,488,802; 7,943,743; 8,008,449; 8,168,757; 8,217,149, and PCT Published Patent Application Nos: WO03042402, WO2008156712, WO2010089411, WO2010036959, WO2011066342, WO2011159877, WO2011082400, and WO2011161699.

In certain options, the blockage agents are selected from anti-PD-L1 antibodies and/or similar binding proteins such as NIVOLUMAB (MDX 1106, BMS 936558, ONO 4538), a fully human IgG4 antibody that binds to and blocks the activation of PD-1 by its ligands PD-L1 and PD-L2; LAMBROLIZUMAB (MK-3475 or SCH 900475), a humanized monoclonal IgG4 antibody against PD-1; CT-011 a humanized antibody that binds PD-1; AMP-224 is a fusion protein of PD-L2 with an antibody Fc portion; BMS-936559 (MDX- 1105-01) for PD-L1 (B7-H1) blockade.

In some options, a test blockade agent (e.g., an agent (e.g., antibody) suspected of inhibiting PD-1/PD-L1 complex formation or with unknown effect on PD-1/PD-L1 complex formation) is administered to a system described herein to test for the potency of the test agent on interrupting the PD-1/PD-L1 interaction. More generally, options herein comprise a test blockade agent (e.g., an agent (e.g., antibody) suspected of inhibiting ICR/ICL complex formation or with unknown effect on ICR/ICL complex formation) is administered to a system described herein to test for the potency of the test agent on interrupting the ICR/ICL interaction.

In some options, described herein are systems and methods for screening test blockade agents for the ability to: (1) inhibit ICR/ICL interactions, (2) activate effector cells, and/or (3) promote transcription from NFAT promoters. In some options, test blockade agent is a compound, peptide, protein, antibody, etc., that is suspected of inhibiting a particular ICR/ICL interaction (e.g., the interaction of PD-1 with PD-L1, PD-1 with PD-L2, CTLA-4 with CD80/CD86, TIGIT with CD155, etc.) or that has demonstrated such inhibiting in another assay. In other options, a test blockade agent has unknown effects on ICR/ICL interactions (e.g., the interaction of PD-1 with PD-L1, PD-1 with PD-L2, CTLA-4 with CD80/CD86, TIGIT with CD155, etc.). Test blockade agents that have not been characterized for effect on ICR/ICL interactions and/or ability to induce effector cell activation may be tested alone or in a screen of multiple test blockade agents (e.g., a high throughput screen) using systems and methods described herein.

In some options, systems and methods are provided for screening multiple test blockade agents of unknown or unconfirmed effect on ICR/ICL interactions (e.g., the interaction of PD-1 with PD-L1, PD-1 with PD-L2, CTLA-4 with CD80/CD86, TIGIT with CD155, etc.). In some options, multiple test blockade agents (e.g., 2, 3, 4, 5, 6, 7, 8, 9, 10, 20, 30, 40, 50, 100, or more, or ranges therein) are introduced into a single blockade assay system described herein. In such options, upon detection of an inhibitory effect on ICR/ICL interactions (e.g., the interaction of PD-1 with PD-L1, PD-1 with PD-L2, CTLA-4 with CD80/CD86, TIGIT with CD155, etc.), as indicated by an increase in reporter signal, individual test blockade agents or smaller groups of the agents will be subsequently tested in separate blockade assay systems (e.g., in separate reaction vessels). In other options, multiple test blockade agents (e.g., 2, 3, 4, 5, 6, 7, 8, 9, 10, 20, 30, 40, 50, 100, 200, 500, 1000, 2000, 5000, 10,000, 20,000, 50,000, 100,000, or more, or ranges therein) are introduced into a parallel blockade assay system described herein. Such parallel assays may be run using systems, methods, devices, robotics, and other equipment known in the art to facilitate large-scale and/or high throughput screens. For example, aAPCs and effector cells are co-cultured in 96- or 384-well plates, and robotics are used to efficiently introduce a library of test blockade agents and to detect reporter signal.

In some options, reagents, compositions, devices, equipment, etc. are provided (e.g., along with the assay components described herein (e.g., effector cells and aAPCs) for carrying out blockade assays. In some options, reagents may include culture media, serum, nutrient supplements (e.g., sufficient for the co-culturing of aAPCs and effector cells), buffers, reporter substrate (e.g., luciferin, coelenterazine (or a derivative thereof), etc.), control blockade agents (e.g., positive controls known to inhibit ICR/ICL interactions (e.g., the interaction of PD-1 with PD-L1, PD-1 with PD-L2, CTLA-4 with CD80/CD86, TIGIT with CD155, etc.)), etc. In some options, culture flasks and vesicles, microcentrifuge tubes, microplates including 96- and 384-well plates, readers, water bath, CO₂ incubator, single channel and multi-channel pipettes, etc., are provided. In some options, robotics for high throughput assays (e.g., regent dispensing, sample organization, reporter detection, etc.), instruments for reporter detection (e.g., fluorometer, luminometer, spectrometer, Taqman, ELISA plates etc.), etc. are provided.

In some options, all or a portion of the components for performing a blockade assay described herein are provided as a kit. The components may be in separate containers that are packaged together or separately for convenience or other considerations. In some options, reagents (e.g., aAPCs, effector cells, etc.) are provided in predetermined amounts according to the size and/or number of blockade assays a user intends to perform. In some options, accessories (e.g., vessels, plates, etc.), instructions, other reagents (e.g., buffers, controls, media, reporter substrate, etc.), etc., are also provided. In some options, a user provides one or more components necessary for using the components (e.g., media, blockade agent(s), reporter substrate, etc.) of the kit to perform a blockade assay described herein.

In some options, methods are provided utilizing the compositions and systems described herein. Methods utilizing the artificial antigen presenting cells (aAPCs) are provided, for example: to screen blockade agents, to develop and further modify and optimize blockade agents, to test the potency of blockade agents, to modulate immunity of effector cells, to identify ICRs and/ICLs, to test immune function in vitro or in vivo, etc. In some options, methods utilize aAPCs in combination with artificial effector cells (e.g., comprising engineered receptors, comprising engineered antigen response elements, comprising engineered reporter constructs, etc.) or natural effector cells (e.g., effector cells from human or animal source comprising only intrinsic components). In some options, methods are performed *in vitro, in vivo, in situ,* in a whole organism (e.g., human or animal model), in cell culture, etc. In some options, methods described herein utilize any of the aAPC, effector cell, blockade agent, or other components described in connection with the compositions and systems herein.

In some options, methods comprise contacting (e.g., co-culturing): (a) an effector cell (e.g., natural or engineered) comprising an ICR, antigen responsive element (e.g., TCR complex), and a reporter (e.g., exogenous or intrinsic) with (b) an aAPC described herein (e.g., displaying an ICL and activator of an antigen responsive element (e.g., TCR activator). In some options, methods further comprise detecting signal from said reporter to quantify: activation of effector cell immunity by the aAPC, modulation of effector cell activation by the formation of an ICR/ICL complex, etc. In some options, methods further comprise contacting the aAPC and/or effector cell with a blockade agent or test blockade agent, and detecting signal from said reporter to quantify the effect of the blockade agent on one or more of: activation of effector cell immunity, formation of an ICR/ICL complex, modulation of effector cell activation by the ICR/ICL complex, an antigen-responsive-element-dependent pathway, an ICR/ICL-dependent pathway, etc.

In some options, described herein are methods comprising: (a) contacting: (i) an artificial antigen presenting cell (aAPC) displaying on its surface: (A) a T cell receptor (TCR) activator and (B) an immune checkpoint ligand (ICL) with (ii) an effector cell comprising a TCR complex and displaying on its surface an immune checkpoint receptor (ICR), wherein interaction of the TCR activator with the TCR complex activates a TCR signaling pathway, wherein said ICR and said ICL form an ICR/ICL complex upon interaction, and wherein formation of the ICR/ICL complex enhances or inhibits TCR activation and/or the TCR signaling pathway; and (b) detecting the presence, absence, and/or level of TCR activation in said effector cell. In some options, methods further comprise: (c) contacting said effector cell and/or said aAPC with a blockade agent or test blockade agent. In some options, methods further comprise: (d) detecting the effect of said blockade agent or test blockade agent on (i) ICR/ICL complex formation, (ii) TCR activation, and/or (iii) the TCR signaling pathway. In some options, one or more of (i) ICR/ICL complex formation, (ii) TCR activation, and/or (iii) the TCR signaling pathway are detected in the presence and absence of said blockade agent or test blockade agent to determine an effect.

In some options, described herein are methods comprising: (a) contacting: (i) an artificial antigen presenting cell (aAPC) displaying on its surface: (A) a T cell receptor (TCR) activator, and (B) an immune checkpoint ligand (ICL), with (ii) an effector cell displaying on its surface an immune checkpoint receptor (ICR) and comprising a TCR complex and reporter (e.g., intrinsic or exogenous), wherein interaction of the TCR activator with the TCR complex activates a TCR signaling pathway, wherein said ICR and said ICL form an ICR/ICL complex upon interaction, wherein formation of the ICR/ICL complex enhances or inhibits TCR activation and/or the TCR signaling pathway, and wherein a detectable signal from said reporter (e.g., activity, luminescence, fluorescence, expression level, concentration, localization, protein-protein interaction, etc.) correlates with the level of TCR activation; and (b) detecting the presence, absence, and/or level of TCR activation in said effector cell. In some options, methods further comprise: (c) contacting said effector cell and/or said aAPC with a blockade agent or test blockade agent. In some options, methods further comprise: (d) detecting the effect of said blockade agent or test blockade agent on (i) ICR/ICL complex formation, (ii) TCR activation, and/or (iii) the TCR signaling pathway. In some options, one or more of (i) ICR/ICL complex formation, (ii) TCR activation, and/or (iii) the TCR signaling pathway are detected in the presence and absence of said blockade agent or test blockade agent to determine an effect.

In some options, aAPCs are introduced into a system (e.g., cell culture system, whole organism, tissue sample, blood sample, etc.), and the interaction of the aAPC and/or a blockade agent with effector cells within the system (or pathways downstream) are assayed (e.g., TCR/TCR-activator interaction, ICL/ICR interaction, disruption of the ICR/ICL interaction by the blockade agent, etc.). In some options, the effector cells are primary cells (e.g., human or animal cells (e.g., isolated or derived from an organism or a cell culture line) with only intrinsic components), and the interaction is assayed based on, for example, a characteristic of an intrinsic reporter within the effector cell (e.g., expression from a TCR-pathway-dependent promoter, activity of a TCR pathway protein, TCR activation-dependent cellular changes, TCR dependent protein-protein interactions and protein movement, or detection of reporter gene regulation by any gene delivery method in transient or stable integration, etc.). In other options, the effector cells are artificial effector cells and comprise a reporter construct for detecting the effects of the aAPC/effect interaction.

In certain options, described herein are methods of assaying the potency of a blockade agent (or test blockade agent) to inhibit the interaction of an ICR and ICL or signaling from an ICR/ICL complex. In such options, a reporter or a signal from a reporter (e.g., natural or engineered) within the effector cell is monitored (e.g., in the presence, absence, and/or varying concentration of blockade agent) to determine and/or quantify the potency of the blockade agent.

In some options, methods of screening (e.g., a library of compounds, peptides, antibodies, etc.) test blockade agents are provided. In such options, a number of substantially identical systems described herein are assayed in parallel to identify the response of the system to a number of test blockade agents. Such assays may be performed in low- or high-throughput formats and may be performed manually or may be automated.

In some embodiment, methods are provided in which the aAPCs described herein are introduced into a biological system (e.g., biological sample (e.g., tissue sample, blood sample, etc.), whole organism, cell culture system, etc.). In some options, the biological system comprises natural effector cells and/or effector cells that have been engineered according to options described herein.

In some options, assays are performed as a service. In such options, a user submits one or more test blockade agents to be tested. One or more blockade assays are performed according to the options described herein, and the results (e.g., inhibitory potency of the test blockade agent) are reported to the user. Test results may be reported in any suitable format and may include raw data (e.g., reporter signal as a function of blockade agent concentration) in an analyzed form to provide the user with readily interpretable results.

Compositions, systems, and methods are described herein which comprise or utilize aAPCs. In some options, such compositions, systems, and methods may instead comprise phospholipid droplets in place of the aAPCs. Such phospholipid droplets display and comprise the same elements as the aAPCs (e.g., TCR activator, immune checkpoint ligand, etc.) described herein and find use in the same methods (e.g., interacting with effector cells).

### EXPERIMENTAL

### Example 1

### PD-1/PD-L1 Blockade Assay Specificity

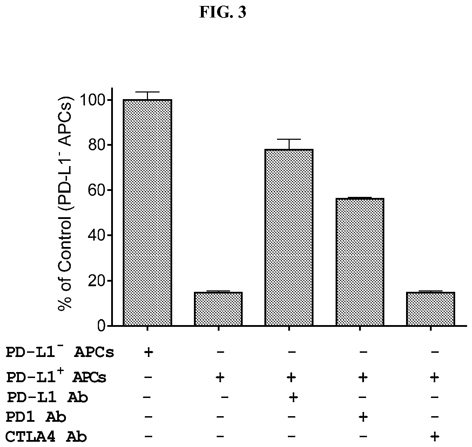

Experiments were conducted during development of options described herein to test the detectable response of the blockade assay depicted in Figure 1 to various conditions. Jurkat effector cells expressing PD-1 and NFAT-RE-luciferase reporter were co-cultured with PD-L1⁻ or PD-L1⁺ aAPC which both display anti-CD3 antibodies (Figure 2 and Figure 3). Luciferase activity significantly decreased in the co-culture system comprising Jurkat effector cells and PD-L1⁺ aAPC compared with co-culture system comprising Jurkat effector cells and PD-L1⁻ aAPC. Different blockage agents were incubated and compared in the system comprising Jurkat effector cells and PD-L1⁺ aAPCs (Figure 3). Only in the presence of specific blockage agent known to specifically blocking the PD-1/PD-L1 interaction, anti-PD-L1 antibody or anti-PD-1 antibody, but not in the presence of anti-CTLA-4 antibody, anti-CD3 antibody activated TCR complex and increased the expression of the luciferase reporter driven by the NFAT response element. Experiments demonstrated the specificity of the system to the various conditions tested.

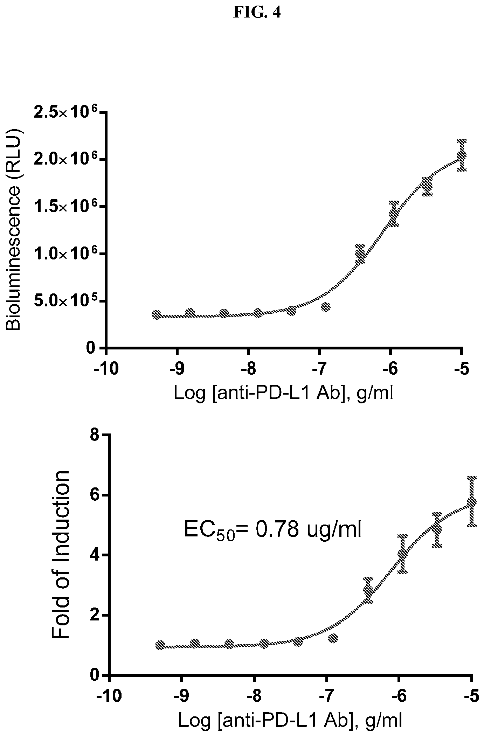

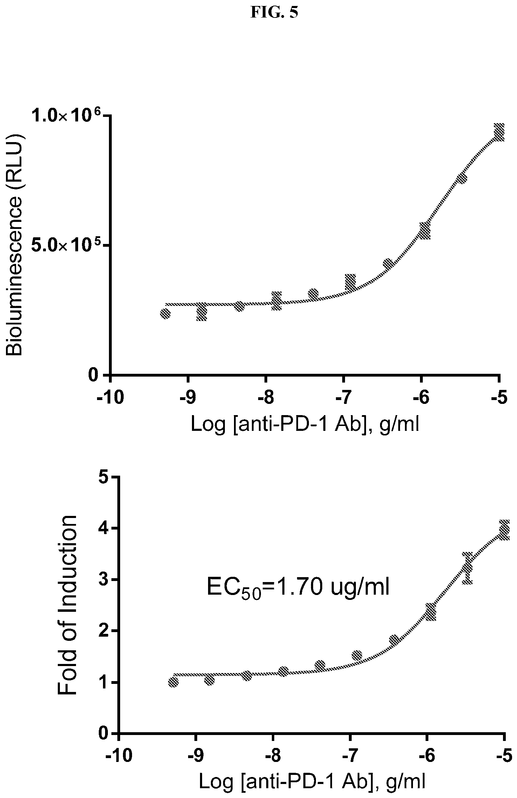

Experiments demonstrated that anti-PD-L1 antibody (Figure 4) and anti-PD-1 antibody (Figure 5) activity can be measured by the reporter system. Jurkat effector cells expressing PD-1 and NFAT-RE-luciferase reporter were co-cultured with anti-CD3/PD-L1⁺ aAPC, in the presence of serial dilution of anti-PD-L1 antibody (Figure 4) or anti-PD-1 antibody (Figure 5). Both blockage agents provided concentration-dependent inhibition of the PD-1/PD-L1 complex formation, resulting in concentration-dependent increase in luminescence signals and increased luciferase activity by 4-6 fold from the reporter system.

### Example 2

### PD-1/PD-L1 Blockade Assay using aAPCs not expressing anti-CD3

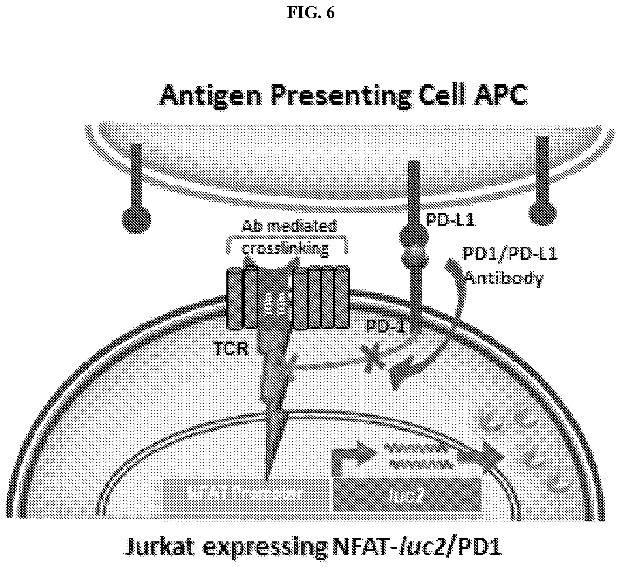

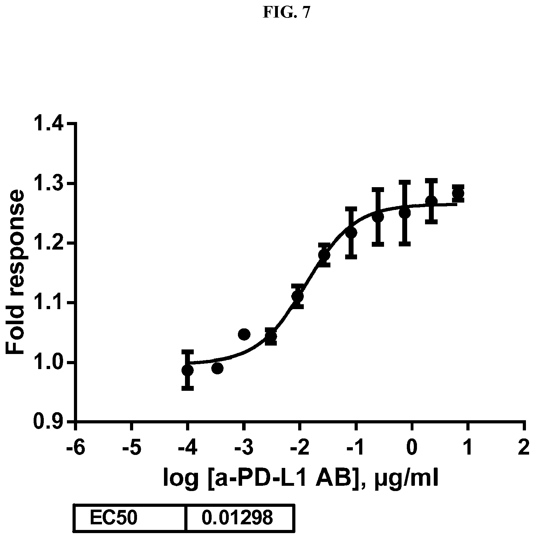

Experiments conducted during development of options described herein demonstrate that blockade assay systems using aAPCs expressing PD-L1, but not expressing anti-CD3, in the presence of soluble anti-CD3 antibody (Figure 6). Jurkat effector cells expressing PD-1 and NFAT-RE-luciferase reporter were co-cultured with anti-CD3⁻/PD-L1⁺ aAPC, in the presence of soluble anti-CD3 antibody and serial dilution of blockage agent, anti-PD-L1 antibody (Figure 6). Blockage agent provided significantly reduced induction (< 1.3 fold) of NFAT-RE-dependent expression of luciferase (Figure 7), compared with 6 fold of increase of luciferase expression from same blockage agent in Figure 4.

### Example 3

### Anti-CD3/PD-Ll beads in place of anti-CD3/PD-Ll aAPCs in Blockade Assay

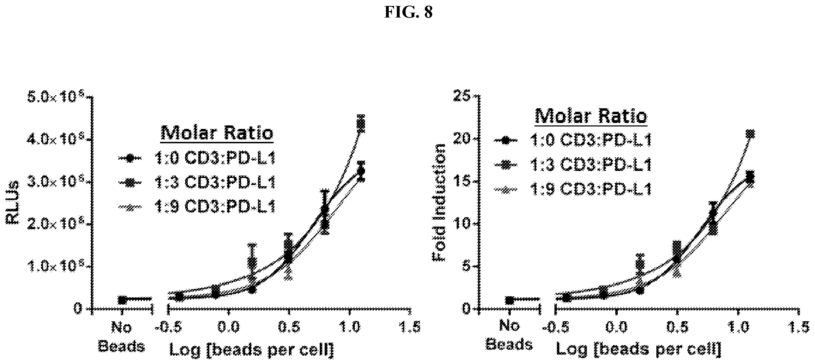

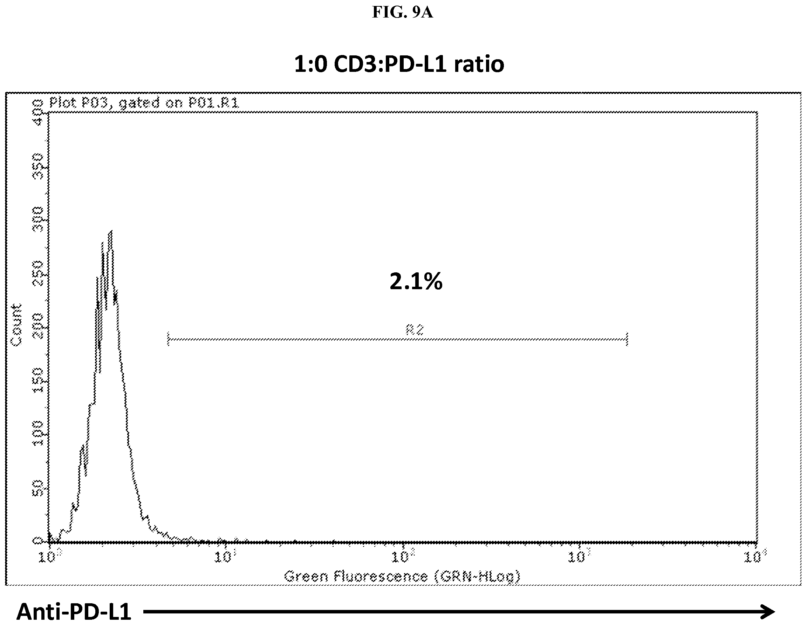

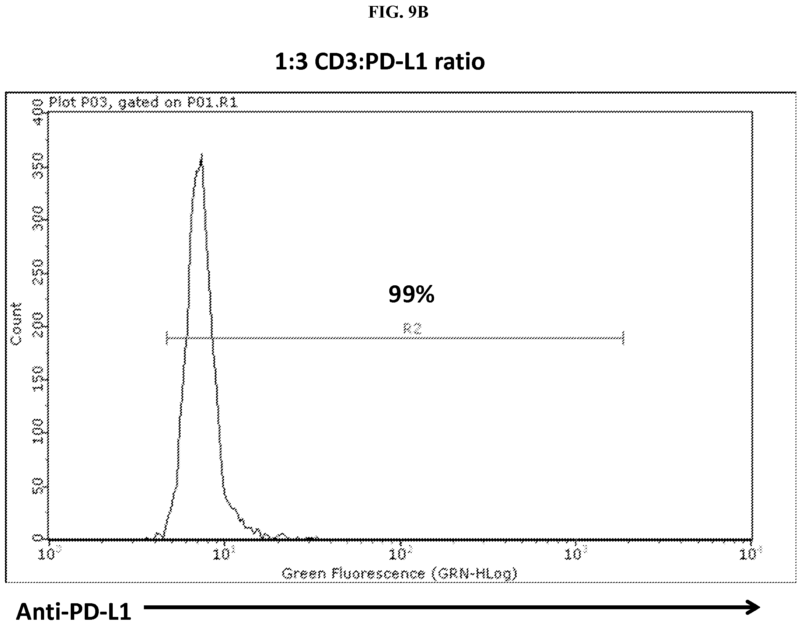

Experiments conducted during development of options described herein demonstrate that anti-CD3/PD-L1 aAPC beads fail to inhibit activation of NFAT-promoter-dependent expression of luciferase by Jurkat effector cells (Figure 8). Beads were coated with a fixed amount of anti-CD3 (10% of protein). PD-L1-Fc chimera was simultaneously coated at 1:0 (no PD-L1), 1:3, and 1:9 molar ratios of anti-CD3:PD-L1-Fc. An irrelevant IgG was used to balance the molar ratios. Jurkat effector cells expressing PD-1 and NFAT-RE-luciferase were incubated with serial bead dilutions. NFAT-RE-dependent luciferase expression was similar whether 1:0, 1:3, or 1:9 anti-CD3:PD-L1-Fc beads were used. FACS analysis confirms the presence of PD-L1 on the beads (Figure 9). Beads described for Figure 8 were labeled with anti-PD-L1-FITC and analyzed by flow cytometry.

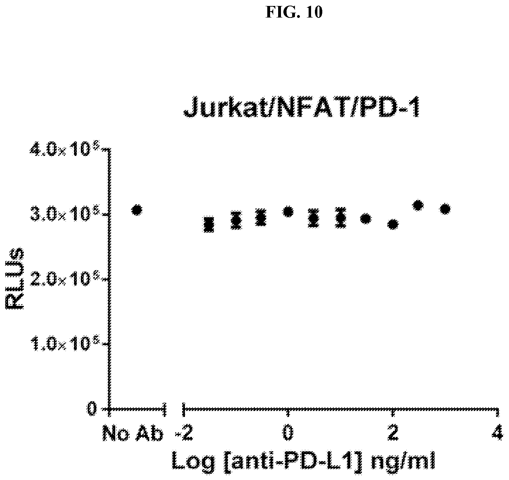

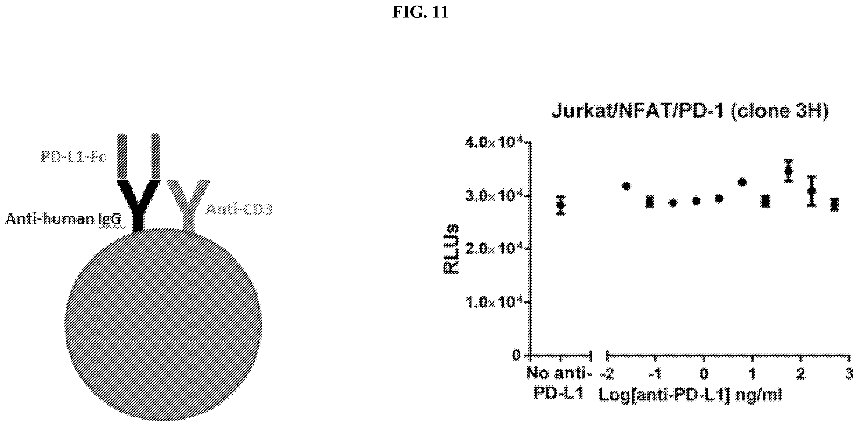

Anti-CD3/PD-L1 beads (1:9 molar ratio) failed to show PD-1/PD-L1 engagement induced inhibition of T cell activation, and anti-PD-L1 antibody failed to induced T cell activation (Figure 10). Jurkat effector cells expressing PD-1 and NFAT-RE-luciferase were treated with anti-CD3:PD-L1-Fc beads in the presence or absence of PD-L1 blocking antibody. NFAT-RE-dependent luciferase expression was unchanged in the presence of PD-L1 blocking antibody. Extended length of PD-L1 on the beads did not remedy the lack of activation by the beads (Figure 11). Shown is an illustration of the strategy to extend PD-L1 away from the bead. Beads were coated with anti-CD3:anti-human IgG at a 1:9 molar ratio. Beads were then incubated with PD-L1-Fc chimera (containing a human IgG1 Fc region fused to PD-L1) to load PD-L1 on the beads. Jurkat effector cells expressing PD-1 and NFAT-RE-dependent luciferase were treated with these beads in the presence or absence of PD-L1 blocking antibody. NFAT-RE-dependent luciferase expression was not affected by the presence of PD-L1 blocking antibody.

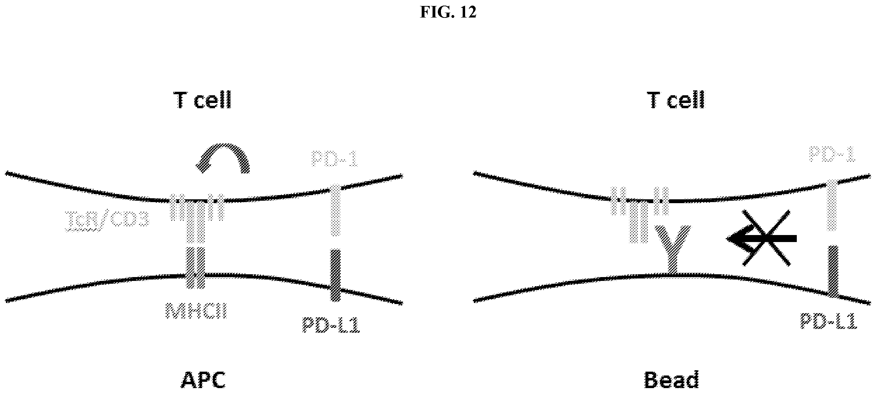

It is contemplated that the rigidity of the placement of anti-CD3 and PD-L1 on the beads may prevent effective T cell inhibition (Figure 12). Specifically, formation of an immune synapse between T cells and aAPCs requires the dynamic lateral localization of membrane-bound receptors and ligands. Upon binding PD-L1, PD-1 is known to be co-localized with the TCR in the immune synapse, where PD-1 can directly inhibit TCR complex signaling. When PD-L1 is localized in the plasma membrane of aAPCs, such lateral localization of PD-1/PD-L1 can be achieved (illustration on the left). When PD-L1 is bound on a bead surface, the position of PD-L1 is static, and lateral movement is restricted (illustration on the right). Thus, PD-1/PD-L1 will not be co-localized with the TCR, and PD-1 will not be able to inhibit TCR complex signaling. This is consistent with published research indicating that PD-1 forms negative co-stimulatory microclusters with the TCR complex, which are required to inhibit proximal TCR signaling (Yokosuka et al. J Exp Med. 2012 Jun 4;209(6):1201-17). The options described herein are not limited to any particular mechanism of action and an understanding of the mechanism of action is not necessary to practice such options.

### Example 4

### PD-1/PD-L2 Blockade Assay Specificity

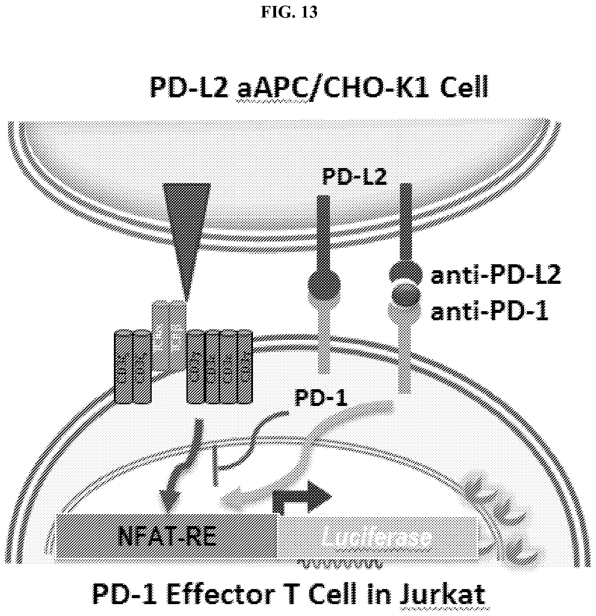

Experiments were conducted during development of options described herein to test the detectable response of the blockade assay depicted in Figure 13 to various conditions. An aAPC displaying PD-L2 and anti-CD3 antibody Fab domain on its cell membrane is shown interacting with a Jurkat T cell displaying PD-1, the TCR complex, and comprising NFAT-promoter-dependent luciferase reporter construct (Jurkat effector cell). Upon the interaction of the aAPC and Jurkat effector cell, anti-CD3 Ab activates the TCR complex and NFAT-promoter; however, the interaction of PD-1 and PD-L2 inhibits TCR activation and luciferase expression from the NFAT promoter, and little expression of the luciferase occurs. In the presence of an inhibitor of the PD-1/PD-L2 interaction, the activated TCR complex activates expression of the luciferase reporter from the NFAT promoter, and luciferase signal is elevated.

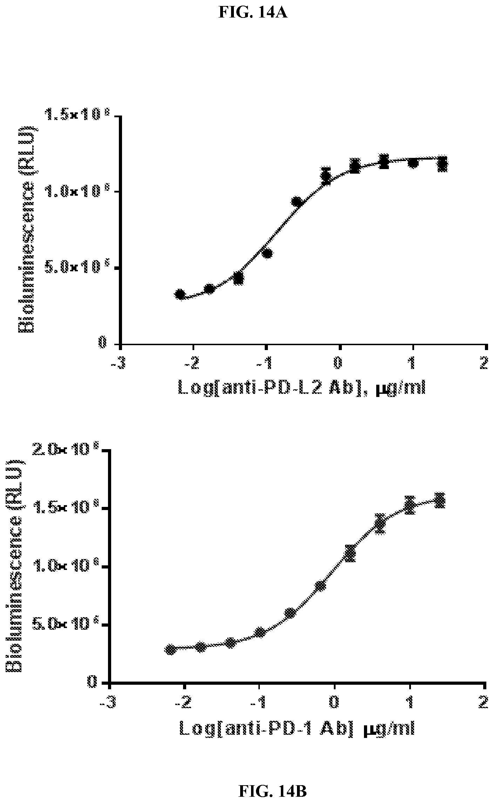

The experiments demonstrated that anti-PD-1 antibody (Figure 14b) and anti-PD-L2 antibody (Figure 14a) activity were measured by the reporter system. Jurkat effector cells expressing PD-1 and NFAT-RE-luciferase reporter were co-cultured with anti-CD3/PD-L2⁺ aAPC, in the presence of serial dilution of anti-PD-L2 antibody (Figure 14a) or anti-PD-1 antibody (Figure 14b). Both blockage agents provided concentration-dependent activation of reporter expression, resulting in concentration-dependent increase in luminescence signals and increased luciferase activity.

### Example 5

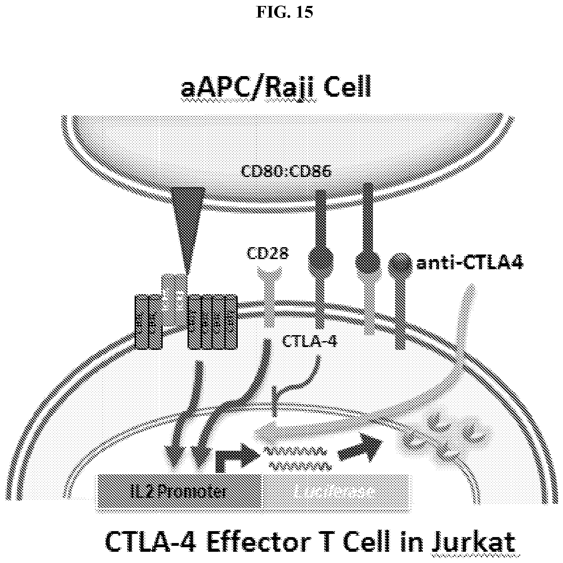

Experiments were conducted during development of options described herein to assess inhibitors of immune checkpoint receptor CTLA-4 and its ligand, CD80/CD86, as depicted in Figure 15. An aAPC in Raji B cells displaying CD80/CD86 and anti-CD3 antibody Fab domain on its cell membrane is shown interacting with a Jurkat T cell displaying co-stimulatory receptor CD28, CTLA-4, the TCR complex, and comprising IL-2-promoter-dependent luciferase reporter construct (Jurkat effector cell). Upon the interaction of the aAPC and Jurkat effector cell, anti-CD3 Ab activates the TCR complex and CD80/CD86 co-stimulates CD28. This leads to activation of the IL-2 promoter, driving luciferase expression; however, CTLA-4 has higher binding affinity with CD80/CD86 than its binding affinity with CD28. It inhibits interaction of CD80/CD86 with CD28 and luciferase expression from the IL-2 promoter. In the presence of an inhibitor of CTLA-4 with CD80/CD86 interaction, the activated CD28 activates expression of the luciferase reporter from IL-2 promoter, and luciferase signal is elevated.

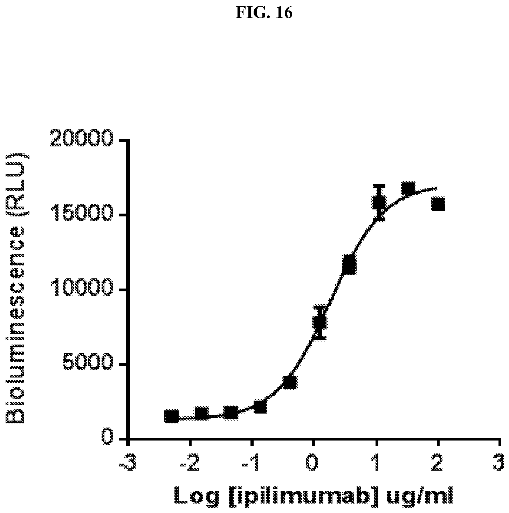

The experiments demonstrated measurement of activity of the anti-CTLA-4 antibody, ipilimumab, by the reporter system (Figure 16). Jurkat effector cells expressing CTLA-4 and an IL-2-luciferase reporter were co-cultured with anti-CD3/CD80/CD86⁺ aAPC, in the presence of a serial dilution of ipilimumab. The blockade agent provided concentration-dependent activation of reporter expression resulting in concentration-dependent increase in luminescence signals and increased luciferase activity.

### Example 6

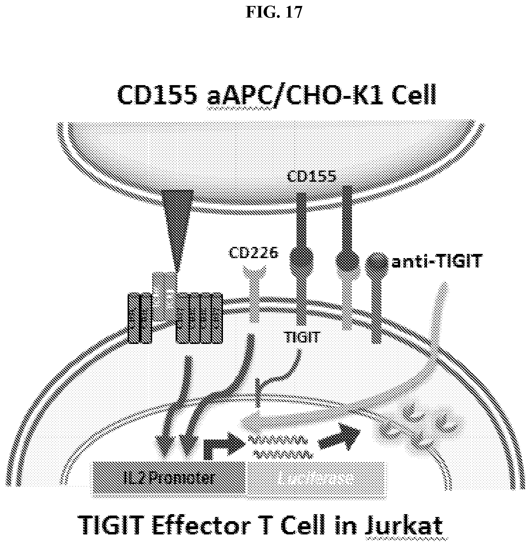

Experiments were conducted during development of options described herein for assessing inhibitors of immune checkpoint receptor TIGIT and its ligand, CD155, as depicted in Figure 17. An aAPC displaying CD155 and anti-CD3 antibody Fab domain on its cell membrane is shown interacting with a Jurkat T cell displaying co-stimulatory receptor CD226, TIGIT, the TCR complex, and comprising IL-2-promoter-dependent luciferase reporter construct. Upon the interaction of the aAPC and Jurkat effector cell, anti-CD3 Ab activates the TCR complex and CD155 co-stimulates CD226. These lead to activation of IL-2 promoter driving luciferase expression; however, CD155 has higher binding affinity with TIGIT than with CD226. Therefore, TIGIT inhibits interaction of CD155 with CD226 and the activation of luciferase expression from the IL-2 promoter. In the presence of an inhibitor of the TIGIT/CD155 interaction, the activated CD226 activates expression of the luciferase reporter from IL-2 promoter, and luciferase signal is elevated.

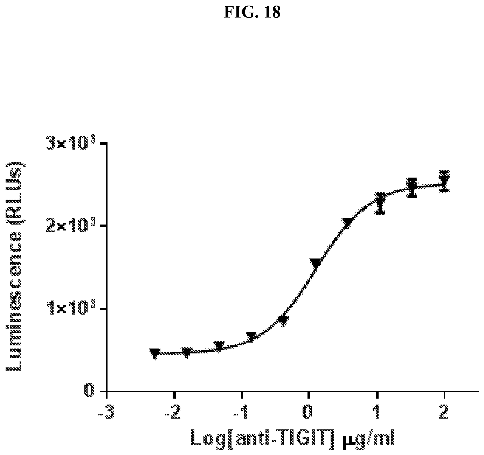

The experiments demonstrated that anti-TIGIT antibody (Figure 18) activity was measured by the reporter system. Jurkat effector cells expressing TIGIT and an IL-2 promoter dependent-luciferase reporter were co-cultured with anti-CD3/CD155 ⁺ aAPC, in the presence of a serial dilution of anti-TIGIT antibody. The blockage agent provided concentration-dependent activation of reporter expression by an anti-TIGIT antibody resulting in an increase in luminescence signals and increased luciferase activity.

### Example 7

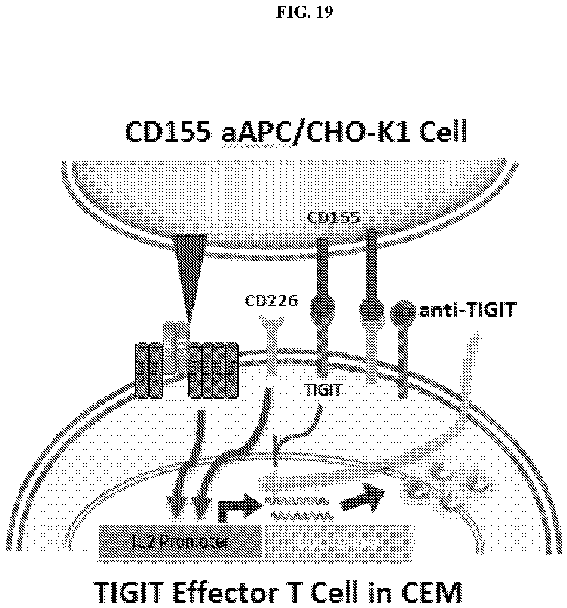

Experiments were conducted during development of options described herein for assessing inhibitors of immune checkpoint receptor TIGIT and its ligand, CD155, as depicted in Figure 19. An aAPC displaying CD155 and anti-CD3 antibody Fab domain on its cell membrane is shown interacting with a CEM T cell displaying co-stimulatory receptor CD226, TIGIT, the TCR complex, and comprising IL-2-promoter-dependent luciferase reporter construct (CEM effector cell). Upon the interaction of the aAPC and CEM effector cell, anti-CD3 Ab activates the TCR complex and CD155 co-stimulates CD226. Both lead to activation of IL-2 promoter driving luciferase expression; however, CD155 has higher binding affinity with TIGIT than with CD226. Therefore, TIGIT inhibits interaction of CD155 with CD226 and the activation of luciferase expression from the IL-2 promoter. In the presence of an inhibitor of the TIGIT/CD155 interaction, the activated CD226 activates expression of the luciferase reporter from IL-2 promoter, and luciferase signal is elevated.

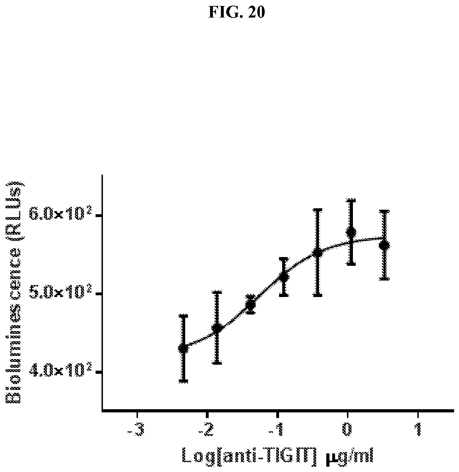

The experiments demonstrated that anti-TIGIT antibody (Figure 20) activity was measured by the reporter system. CEM T effector cells expressing TIGIT and an IL-2 promoter dependent-luciferase reporter were co-cultured with anti-CD3/CD155 ⁺ aAPC, in the presence of a serial dilution of anti-TIGIT antibody. The blockage agent provided concentration-dependent activation of reporter expression by an anti-TIGIT antibody resulting in an increase in luminescence signals and increased luciferase activity.

### Example 8

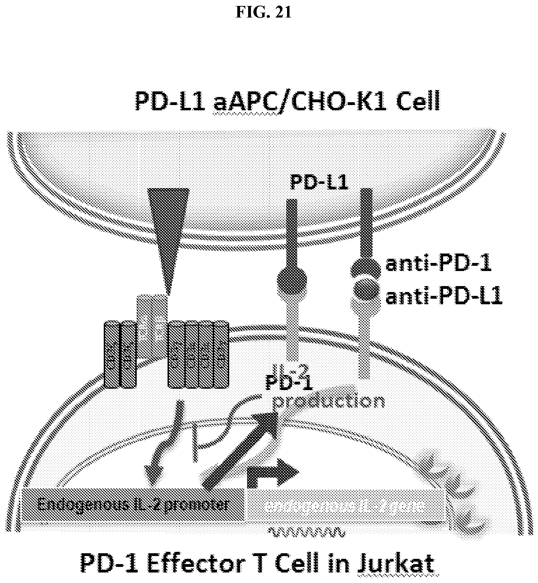

Experiments were conducted during development of options described herein for assessing inhibitors of immune checkpoint receptor PD-1 and its ligand PD-L1, as depicted in Figure 21. An aAPC displaying PD-L1 and anti-CD3 antibody Fab domain on its cell membrane is shown interacting with a Jurkat T cell displaying PD-1, the TCR complex, and endogenous IL-2 gene driven by native IL-2 promoter. Upon the interaction of the aAPC and Jurkat effector cell, anti-CD3 Ab activates the TCR complex, activates IL-2 promoter and increases IL-2 production; however, the interaction of PD-1 and PD-L1 inhibits activation of IL-2 expression from IL-2 promoter, and little production of IL-2 proteins occurs. In the presence of an inhibitor of the PD-1/PD-L1 interaction, the activated TCR complex activates IL-2 expression from IL-2 promoter, and IL-2 production in the media is elevated.

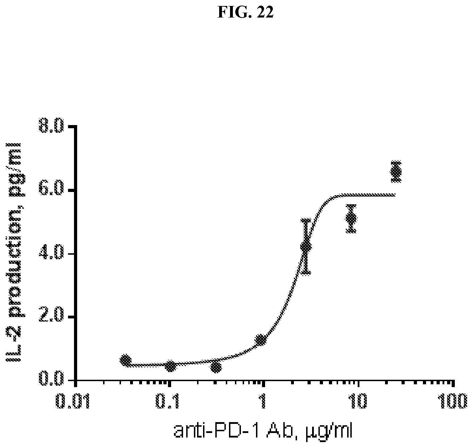

The experiments demonstrated that anti-PD-1 antibody (Figure 22) activity was measured by ELISA. Jurkat T effector cells expressing PD-1 and an endogenous IL-2 gene driven by native IL-2 promoter were co-cultured with anti-CD3/PD-L1 ⁺ aAPC, in the presence of a serial dilution of anti-PD-1 antibody. The blockage agent provided concentration-dependent IL-2 production by an anti-PD-1 antibody.

### Example 9

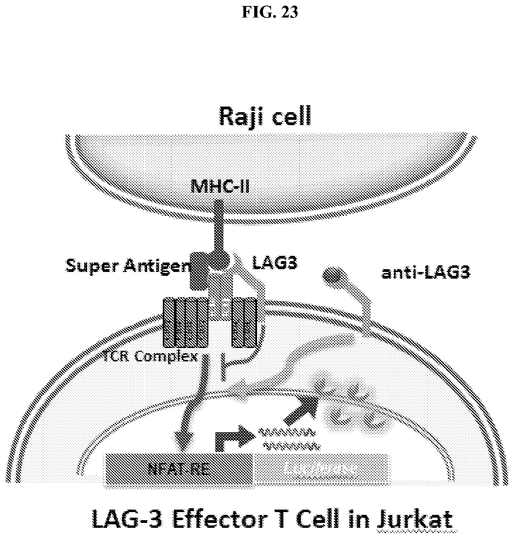

Experiments were conducted during development of options described herein for assessing inhibitors of immune checkpoint receptor LAG-3 and its ligand, MHC II, as depicted in Figure 23. An APC in Raji B cells displaying MHC II on its cell membrane is shown interacting with a Jurkat T cell displaying LAG-3, the TCR complex, and comprising NFAT-promoter-dependent luciferase reporter construct (Jurkat effector cells). Upon the interaction of the APC and Jurkat effector cell, super antigen presented by MHC II activates the TCR complex and activation of NFAT promoter driving luciferase expression; however, LAG-3 binding with MHC II inhibits TCR activation. This leads to inactivation of luciferase expression from the NFAT promoter. In the presence of an inhibitor of LAG-3/MHC II interaction, the activated TCR activates expression of the luciferase reporter from NFAT promoter, and luciferase signal is elevated.

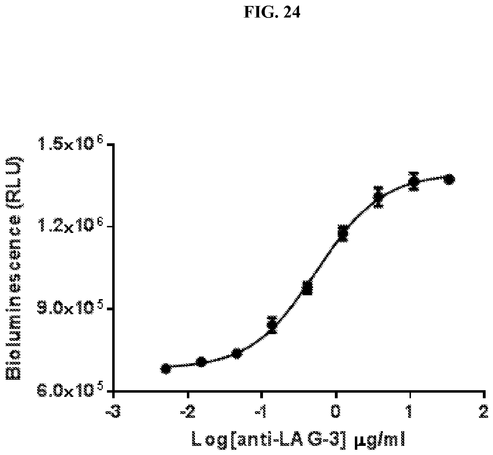

The experiments demonstrated that anti-LAG-3 antibody (Figure 24) activity was measured by the reporter system. Jurkat effector cells expressing LAG-3 and NFAT RE-luciferase reporter were co-cultured with anti-MHC II⁺ aAPC, in the presence of a serial dilution of anti-LAG-3 antibody. The blockage agent provided concentration-dependent activation of reporter expression by an anti-LAG-3 antibody resulting in an increase in luminescence signals and increased luciferase activity.

### Example 10

Experiments were conducted during development of options described herein for assessing inhibitors of immune checkpoint receptors TIGIT and PD-1 and their ligands, CD155 and PD-L1, respectively, as shown in Figure 25. An aAPC displaying PD-L1, CD155, and anti-CD3 antibody Fab domain on its cell membrane is shown interacting with a Jurkat T cell displaying PD-1, co-stimulatory receptor CD226, TIGIT, the TCR complex, and comprising IL2-promoter-dependent luciferase reporter construct. Upon the interaction of the aAPC and Jurkat effector cell, anti-CD3 Ab activates the TCR complex and CD155 co-stimulates CD226. These led to activation of IL-2 promoter driving luciferase expression; however, the interaction of PD-1 with PD-L1 leads to reduced TCR pathway activation and reduced IL-2 promoter-dependent reporter expression. In addition, CD155 has higher binding affinity with TIGIT than with CD226. Therefore, TIGIT inhibits interaction of CD155 with CD226 and leads to reduced reporter expression. In the presence of an inhibitor of the PD-1/PD-L1 and/or TIGIT/CD155 interaction, the expression of the luciferase reporter from IL-2 promoter is enhanced, and the luciferase signal is elevated.

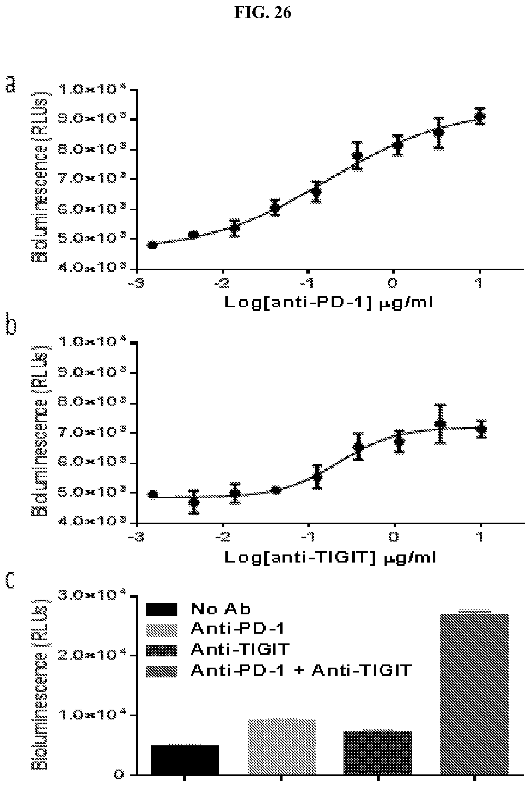

Figure 26a-b shows graphs depicting concentration-dependent activation of reporter expression by (a) an anti-PD-1 antibody and (b) an anti-TIGIT antibody in an exemplary PD-1/TIGIT blockade assay using a PD-1/TIGIT effector Cells in Jurkat-T cells and PD-L1/CD155 aAPC/CHO-K1 cells. Figure 26c shows the effects of a combination of anti-PD-1 and anti-TIGIT antibodies compared with either antibody alone.

## Claims

1. A composition comprising:
(a) an effector cell displaying on its surface (i) a T cell receptor (TCR) and (ii) an immune checkpoint receptor (ICR) comprising an immune inhibitory receptor selected from the group consisting of: PD-1, CTLA-4, LAG-3, TIM-3, TIGIT, CD160, BTLA, and IL-10 receptor, wherein the effector cell comprises a gene encoding a fluorescent or bioluminescent reporter protein, the expression of which is under control of a nuclear factor of activated T-cells (NFAT) promoter, wherein the NFAT promoter comprises one or more NFAT response elements having at least 50% sequence identity to SEQ ID NO:1, and wherein the NFAT response elements bind to an NFAT family transcription factor to enhance transcription of associated genes; and
(b) an antigen presenting cell (APC) displaying on its surface: (i) a T cell receptor (TCR) activator and (ii) an immune checkpoint ligand (ICL) selected from the group consisting of PD-L1, PD-L2, B7-H4, CD155, galectin-9, and HVEM, wherein the TCR activator is an anti-cluster-of-differentiation-3 (CD3) antibody; wherein the ICR and ICL form an ICR/ICL complex upon interaction, and wherein formation of the ICR/ICL complex results in altered expression of the fluorescent or bioluminescent reporter protein.

2. The composition of claim 1, wherein the effector cell is selected from T cells including Jurkat, HuT-78, CEM, Molt-4 and primary T cells.

3. The composition of claim 2, wherein formation of the ICR/ICL complex results in modulation of TCR activation by the TCR activator and/or modulation of one or more TCR-dependent pathways, optionally
wherein modulation comprises:
(a) inhibition of TCR activation by the TCR activator and/or one or more TCR-dependent pathways; or
(b) enhancement of TCR activation by the TCR activator and/or one or more TCR-dependent pathways.

4. The composition of any one of claims 1 to 3, wherein:
(i) the TCR-pathway promoter is nuclear factor of activated T-cells (NFAT) promoter; or,
(ii) the bioluminescent protein is a luciferase.

5. The composition of claim 1 wherein the effector cell comprises TCR and PD-1 on its surface, and an NFAT-promoter-dependent luciferase; and the antigen presenting cell (APC) displaying on its surface: anti-CD3 antibody and PD-L1 wherein formation of a PD-1/PD-L1 complex results in a suppressed expression of the NFAT-promoter-dependent luciferase.

6. The composition of claim 5, wherein the system further comprises a blockade agent or test blockade agent, wherein the blockade agent or test blockade agent is a small molecule, peptide, protein, or antibody that inhibits formation of the PD-1/PD-L1 complex, resulting in an increased expression of the NFAT-promoter-dependent luciferase.

7. A method for assessing modulators of immune checkpoints, said method comprising:
(a) producing a composition as defined in any one of claims 1 to 6, wherein production of the composition comprises co-culturing an effector cell defined in any one of claims 1 to 6 and an antigen-presenting cell defined in any one of claims 1 to 6; and
(b) detecting a fluorescent or bioluminescent signal from said fluorescent or bioluminescent reporter protein.

8. The method of claim 7, further comprising adding to the composition a blockade agent, wherein the blockade agent is a small molecule, peptide, protein, or antibody that inhibits formation of the ICR/ICL complex or inhibits ICR/ICL-dependent modulation of TCR activation by the TCR activator and/or modulation of one or more TCR-dependent pathways.

9. The method of claim 8, wherein said TCR-pathway-dependent reporter or a signal from said reporter is detected: (1) before, (2) concurrent with, and/or (3) after addition of said blockade agent, optionally wherein the method further comprises a step of comparing signal from (1) before, (2) concurrent with, and/or (3) after addition of said blockade agent to determine the effect of the blockade agent.

10. The method of claim 8, further comprising:
(c) adding a test blockade agent;
(d) detecting said signal from said reporter; and
(e) comparing said signal from said reporter with a control, wherein a gain of signal with respect to the control indicates inhibition of said ICR/ICL complex by said test blockade agent.

11. The method of claim 8, further comprising:
(c) detecting a signal from said reporter;
(d) adding a test blockade agent;
(e) repeating detection of said signal from said reporter; and
(f) comparing said signal from step (c) with said signal from step (e), wherein a gain of signal from step (c) to step (e) indicates inhibition of said ICR/ICL complex by said test blockade agent.

12. The method of claim 8, further comprising:
(c) adding said test blockade agent; and
(d) repeating detection of said signal from said reporter,
optionally further comprising:
(e) comparing said signal from step (b) with said signal from step (d), wherein a gain of signal from step (b) to step (d) indicates inhibition of said ICR/ICL complex by said test blockade agent.

## Patentansprüche

1. Zusammensetzung, die Folgendes umfasst:
(a) eine Effektorzelle, die auf ihrer Oberfläche (i) einen T-Zell-Rezeptor (T cell receptor - TCR) und (ii) einen Immun-Checkpoint-Rezeptor (ICR) darstellt, der einen immunhemmenden Rezeptor umfasst, ausgewählt aus der Gruppe, die aus Folgendem besteht: PD-1, CTLA-4, LAG-3, TIM-3, TIGIT, CD160, BTLA und IL-10-Rezeptor, wobei die Effektorzelle ein Gen umfasst, das ein fluoreszentes oder ein biolumineszentes Reporterprotein codiert, dessen Expression von einem Promotor eines Kernfaktors aktivierter T-Zellen (nuclear factor of activated T-cells - NFAT) kontrolliert wird, wobei der NFAT-Promotor ein oder mehrere NFAT-Antwortelemente umfasst, die wenigstens 50 % Sequenzidentität zu SEQ ID NO: 1 aufweisen, und wobei die NFAT-Antwortelemente an einen Transkriptionsfaktor der NFAT-Familie binden, um eine Transkription assoziierter Gene zu verbessern; und
(b) eine antigenpräsentierende Zelle (antigen presenting cell - APC), die auf ihrer Oberfläche Folgendes darstellt: (i) einen Aktivator eines T-Zell-Rezeptors (TCR) und (ii) einen Immun-Checkpoint-Liganden (ICL), ausgewählt aus der Gruppe, die aus PD-L1, PD-L2, B7-H4, CD155, Galectin-9 und HVEM besteht, wobei der TCR-Aktivator ein Antikörper von Anti-Cluster-of-Differentiation-3 (CD3) ist; wobei der ICR und der ICL bei Interaktion einen ICR/ICL-Komplex ausbilden und
wobei die Ausbildung des ICR/ICL-Komplexes zu einer veränderten Expression des fluoreszenten oder biolumineszenten Reporterproteins führt.

2. Zusammensetzung nach Anspruch 1, wobei die Effektorzelle aus T-Zellen ausgewählt ist, die Jurkat, HuT-78, CEM, Molt-4 und primäre T-Zellen einschließen.

3. Zusammensetzung nach Anspruch 2, wobei die Ausbildung des ICR/ICL-Komplexes zu einer Modulation der TCR-Aktivierung durch den TCR-Aktivator und/oder einer Modulation eines oder mehrerer TCR-abhängiger Stoffwechselwege führt, optional wobei die Modulation Folgendes umfasst:
(a) Hemmung der TCR-Aktivierung durch den TCR-Aktivator und/oder einen oder mehrere TCR-abhängige Stoffwechselwege; oder
(b) Verbesserung der TCR-Aktivierung durch den TCR-Aktivator und/oder einen oder mehrere TCR-abhängige Stoffwechselwege.

4. Zusammensetzung nach einem der Ansprüche 1 bis 3, wobei:
(i) der TCR-Stoffwechselweg-Promotor ein Promotor eines Kernfaktors von aktivierten T-Zellen (NFAT) ist; oder,
(ii) das biolumineszente Protein eine Luciferase ist.

5. Zusammensetzung nach Anspruch 1, wobei die Effektorzelle TCR und PD-1 auf ihrer Oberfläche und eine NFAT-Promotor abhängige Luciferase umfasst; und wobei die antigenpräsentierende Zelle (APC) auf ihrer Oberfläche Folgendes darstellt: Anti-CD3-Antikörper und PD-L1, wobei die Ausbildung eines PD-1/PD-L1-Komplexes zu einer unterdrückten Expression der NFAT-Promotor abhängigen Luciferase führt.

6. Zusammensetzung nach Anspruch 5, wobei das System ferner ein Blockademittel oder ein Testblockademittel umfasst, wobei das Blockademittel oder das Testblockademittel ein kleines Molekül, ein Peptid, ein Protein oder ein Antikörper ist, das/der die Ausbildung des PD-1/PD-L1-Komplexes hemmt, was zu einer erhöhten Expression der NFAT-Promotor abhängigen Luciferase führt.

7. Verfahren zum Bewerten von Modulatoren von Immun-Checkpoints, wobei das Verfahren Folgendes umfasst:
(a) Herstellen einer Zusammensetzung nach einem der Ansprüche 1 bis 6, wobei die Herstellung der Zusammensetzung ein Co-Kultivieren einer Effektorzelle, die in einem der Ansprüche 1 bis 6 definiert ist, und einer antigenpräsentierenden Zelle, die in einem der Ansprüche 1 bis 6 definiert ist, umfasst; und
(b) Erfassen eines fluoreszenten oder biolumineszenten Signals aus dem fluoreszenten oder biolumineszenten Reporterprotein.

8. Verfahren nach Anspruch 7, das ferner ein Zugeben eines Blockademittels zu der Zusammensetzung umfasst, wobei das Blockademittel ein kleines Molekül, ein Peptid, ein Protein oder ein Antikörper ist, das/der die Ausbildung des ICR/ICL-Komplexes hemmt oder die ICR/ICL-abhängige Modulation von TCR-Aktivierung durch den TCR-Aktivator und/oder Modulation eines oder mehrerer TCR-abhängiger Stoffwechselwege hemmt.

9. Verfahren nach Anspruch 8, wobei der Reporter TCR-abhängiger Stoffwechselwege oder ein Signal von dem Reporter: (1) vor, (2) gleichzeitig mit und/oder (3) nach Zugabe des Blockademittels erfasst wird, optional wobei das Verfahren ferner einen Schritt eines Vergleichs des Signals von (1) vor, (2) gleichzeitig mit und/oder (3) nach Zugabe des Blockademittels umfasst, um die Wirkung des Blockademittels zu bestimmen.

10. Verfahren nach Anspruch 8, das ferner Folgendes umfasst:
(c) Zugeben eines Testblockademittels;
(d) Erfassen des Signals von dem Reporter; und
(e) Vergleichen des Signals von dem Reporter mit einer Kontrolle, wobei eine Signalverstärkung in Bezug auf die Kontrolle eine Hemmung des ICR/ICL-Komplexes durch das Testblockademittel anzeigt.

11. Verfahren nach Anspruch 8, das ferner Folgendes umfasst:
(c) Erfassen eines Signals von dem Reporter;
(d) Zugeben eines Testblockademittels;
(e) Wiederholen des Erfassens des Signals von dem Reporter; und
(f) Vergleichen des Signals aus Schritt (c) mit dem Signal aus Schritt (e), wobei eine Signalverstärkung von Schritt (c) zu Schritt (e) eine Hemmung des ICR/ICL-Komplexes durch das Testblockademittel anzeigt.

12. Verfahren nach Anspruch 8, das ferner Folgendes umfasst:
(c) Zugeben des Testblockademittels; und
(d) Wiederholen des Erfassens des Signals von dem Reporter, das optional ferner Folgendes umfasst:
(e) Vergleichen des Signals aus Schritt (b) mit dem Signal aus Schritt (d), wobei eine Signalverstärkung von Schritt (b) zu Schritt (d) eine Hemmung des ICR/ICL-Komplexes durch das Testblockademittel anzeigt.

## Revendications

1. Composition comprenant :
(a) une cellule effectrice manifestant sur sa surface (i) un récepteur de cellules T (TCR) et (ii) un récepteur de point de contrôle immunitaire (ICR) comprenant un récepteur immunosuppresseur choisi dans le groupe constitué par : PD-1, CTLA-4, LAG- 3, TIM-3, TIGIT, CD160, BTLA et le récepteur IL-10, la cellule effectrice comprenant un gène codant pour une protéine rapporteur fluorescente ou bioluminescente, dont l'expression est sous le contrôle d'un promoteur du facteur nucléaire des cellules T activées (NFAT), le promoteur NFAT comprenant un ou plusieurs éléments de réponse NFAT ayant au moins 50 % d'identité de séquence avec SEQ ID NO : 1, et les éléments de réponse NFAT se liant à un facteur de transcription de la famille NFAT pour améliorer la transcription des gènes associés ; et
(b) une cellule de présentation de l'antigène (APC) manifestant sur sa surface : (i) un activateur du récepteur de cellules T (TCR) et (ii) un ligand de point de contrôle immunitaire (ICL) choisi dans le groupe constitué par PD-L1, PD-L2, B7-H4, CD155, galectine-9 et HVEM, l'activateur du TCR étant un anti cluster d'anticorps de différentiation 3 (CD3) ; l'ICR et l'ICL formant un complexe ICR / ICL lors de l'interaction, et la formation
du complexe ICR / ICL entraînant une expression modifiée de la protéine rapporteuse fluorescente ou bioluminescente.

2. Composition selon la revendication 1, dans laquelle la cellule effectrice est choisie parmi les cellules T comportant Jurkat, HuT-78, CEM, Molt-4 et les cellules T primaires.

3. Composition selon la revendication 2, dans laquelle la formation du complexe ICR / ICL entraîne une modulation de l'activation du TCR par l'activateur du TCR et/ou une modulation d'une ou de plusieurs voies dépendantes du TCR, éventuellement
la modulation comprenant :
(a) l'inhibition de l'activation du TCR par l'activateur du TCR et/ou une ou plusieurs voies dépendantes du TCR ; ou
(b) l'amélioration de l'activation du TCR par l'activateur du TCR et/ou une ou plusieurs voies dépendantes du TCR.

4. Composition selon l'une quelconque des revendications 1 à 3, dans laquelle :
(i) le promoteur de la voie du TCR est le promoteur du facteur nucléaire des cellules T activées (NFAT) ; ou,
(ii) la protéine bioluminescente est une luciférase.

5. Composition selon la revendication 1, dans laquelle la cellule effectrice comprend TCR et PD-1 sur sa surface, et une luciférase dépendante du promoteur NFAT ; et la cellule de présentation de l'antigène (APC) manifestant sur sa surface : un anticorps anti-CD3 et PD-L1, la formation d'un complexe PD-1 / PD-L1 entraînant une suppression de l'expression de la luciférase dépendante du promoteur NFAT.

6. Composition selon la revendication 5, dans laquelle le système comprend en outre un agent de blocage ou un agent de blocage de test, l'agent de blocage ou l'agent de blocage de test étant une petite molécule, un peptide, une protéine ou un anticorps qui inhibe la formation du complexe PD-1 / PD-L1, entraînant une expression accrue de la luciférase dépendante du promoteur NFAT.

7. Procédé d'évaluation des modulateurs de points de contrôle immunitaire, ledit procédé comprenant :
(a) la production d'une composition selon l'une quelconque des revendications 1 à 6, la production de la composition comprenant la co-culture d'une cellule effectrice définie dans l'une quelconque des revendications 1 à 6 et d'une cellule de présentation de l'antigène définie dans l'une quelconque des revendications 1 à 6 ; et
(b) la détection d'un signal fluorescent ou bioluminescent provenant de ladite protéine rapporteuse fluorescente ou bioluminescente.

8. Procédé selon la revendication 7, comprenant en outre l'ajout à la composition d'un agent de blocage, dans lequel l'agent de blocage est une petite molécule, un peptide, une protéine ou un anticorps qui inhibe la formation du complexe ICR / ICL ou inhibe la modulation dépendante de l'ICR / ICL de l'activation du TCR par l'activateur du TCR et/ou la modulation d'une ou plusieurs voies dépendantes du TCR.

9. Procédé selon la revendication 8, dans lequel ledit rapporteur dépendant de la voie du TCR ou un signal dudit rapporteur est détecté : (1) avant, (2) simultanément avec, et/ou (3) après l'ajout dudit agent de blocage, éventuellement dans lequel le procédé comprend en outre une étape de comparaison du signal provenant de (1) avant, (2) simultanément avec et/ou (3) après l'ajout dudit agent de blocage pour déterminer l'effet de l'agent de blocage.

10. Procédé selon la revendication 8, comprenant en outre :
(c) l'ajout d'un agent de blocage de test ;
(d) la détection dudit signal provenant dudit rapporteur ; et
(e) la comparaison dudit signal à partir dudit rapporteur avec un témoin, un gain de signal par rapport au témoin indiquant l'inhibition dudit complexe ICR / ICL par ledit agent de blocage de test.

11. Procédé selon la revendication 8, comprenant en outre :
(c) la détection d'un signal à partir dudit rapporteur ;
(d) l'ajout d'un agent de blocage de test ;
(e) la répétition de la détection dudit signal provenant dudit rapporteur ; et
(f) la comparaison dudit signal de l'étape (c) avec ledit signal de l'étape (e), un gain de signal de l'étape (c) à l'étape (e) indiquant l'inhibition dudit complexe ICR / ICL par ledit agent de blocage de test.

12. Procédé selon la revendication 8, comprenant en outre :
(c) l'ajout dudit agent de blocage de test ; et
(d) la répétition de la détection dudit signal provenant dudit rapporteur, comprenant en outre éventuellement :
(e) la comparaison dudit signal de l'étape (b) avec ledit signal de l'étape (d), un gain de signal de l'étape (b) à l'étape (d) indiquant l'inhibition dudit complexe ICR / ICL par ledit agent de blocage de test.


### SEQUENCE LISTING

<110> PROMEGA CORPORATION
<120> SYSTEMS AND METHODS FOR ASSESSING MODULATORS OF IMMUNE CHECKPOINTS
<130> PRMG-34124/WO-1/ORD
<150> US 62/082,458
   <151> 2014-11-20
<160> 1
<170> PatentIn version 3.5
<210> 1
   <211> 30
   <212> DNA
   <213> Artificial sequence
<220>
   <223> NFAT response element
<400> 1
   ggaggaaaaa ctgtttcata cagaaggcgt 30